# EUROPEAN PATENT APPLICATION

(11) **EP 3 135 771 A1**
(43) Date of publication of application: **01.03.2017**
(21) Application number: 15306324.3
(22) Date of filing: 27.08.2015
(51) Int. Cl.: C12Q 1/68

(54) **METHOD FOR EVALUATING INDIVIDUAL RADIOSENSITIVITY AND THE RISK OF ADVERSE EFFECTS**

(71) Applicant: COMMISSARIAT A L'ENERGIE ATOMIQUE ET AUX ENERGIES ALTERNATIVES, 75015 Paris (FR)
(72) Inventor: Schmitz, Annette, 92260 Fontenay aux Roses (FR); Baijer, Jan, 92260 Fontenay aux Roses (FR); Azria, David, 30250 Fontanes (FR); Kerns, Sarah, New York, NY 10029-5674 (US); Perdry, Hervé, 92120 Montrouge (FR)
(74) Representative: Cabinet Becker et Associés

(57) **Abstract**

The present invention relates to a single nucleotide polymorphism (SNP) rs3815496 or any SNP having 100 % of linkage disequilibrium with this SNP as a biomarker for assessing the risk of developing adverse effects after radiotherapy in a subject, in particular acute and/or subacute dermatitis and lymphocyte apoptosis.

SEQUENCE LISTING

<110>  COMMISSARIAT A L'ENERGIE ATOMIQUE ET AUX ENERGIES
       ALTERNATIVES ET AL

<120>  METHOD FOR EVALUATING INDIVIDUAL RADIOSENSITIVITY AND THE RISK OF
        ADVERSE EFFECTS

<130>  B2081EP

<160>  24

<170>  PatentIn version 3.5

<210>  1
<211>  1001
<212>  DNA
<213>  artificial sequence

<220>
<223>  rs3815496

<220>
<221>  misc_feature
<222>  (501)..(501)
<223>  r is a or g

<400>  1
cctcctggat tcacaccatt ctcctgcctc agcctcctga gtagttggga ctataggtgc      60

ctgccaccat gcccagctaa tttttttgta tttttagtag agatggggtt tcaccgtgtt     120

agccaggata gtctcaatct cctgaccatc tgtttgttgg taccactctg tttatttctg     180

tatgtaattc aaggtggaaa tttgccaata cgtagttatt tgcacaattt aactcagcag     240

gattattaca cagctacagc tatttcttta tttatatttt tggaaaaggg atagaatctt     300

cactttgttg tatcttcact ttgtttctct ttggacccta cccgtgagac actaataaat     360

tataatccat gttactgctt attctttccg tgtaaagtac ttttcaagtt gatcataagc     420

tgaggtaggt tgatcataat atcaaaatgt tatttttatg aaaactcata gaatcaactt     480

tggaagccca gtatgcttac ratggctgat tgacaacacc tatgaagagc aaggtattgg     540

agaactagtt tggcaagtgt tgatgaccct ttatcatttc tcttttagaa aagcaacaaa     600

atatttctcc cctagtgaga gaaagaggtc ctcagagagt agcagctcac ataactggga     660

ccagaggaag aagcaacaca ttgtcttctc caagtaagag aaacaacaaa taagtgactt     720

aagggaaaat aatggaagga ttgtgccaag taacttattt tttaaagaat tttatctgaa     780

atgtttatat ggctatctac aaaatgctca gtattaatca gatctcagat tgactgtttt     840

cagatgcaag attacagaca aacagctcaa ttattgccac gacataactg gcctcaggaa     900

aattacaaag tgggagaatt ttgtctttgc agttttcagg atcccatttt ttttccttct     960

ttagagtaat ttctcgctct ttctttcttt ctttttcttt c                        1001

<210>  2
<211>  1001
<212>  DNA
<213>  artificial sequence

<220>
<223>  rs1131532


<220>
<221>  misc_feature
<222>  (501)..(501)
<223>  b is c, g or t

<400>  2
acaaaaatat aggctggcca cagctgcaaa aaagctcctt gctttgctac aaattccctt      60

ctgttaacgc tctttctctc taaatttcct acagactcca agaatgaaaa ggctctgggc     120

cgcaaaataa actcctggga atcatcaagg agtgggcatt cattcctgag caacttgcac     180

ttgaggaatg gtgaactggt catccatgaa aaagggtttt actacatcta ttcccaaaca     240

tactttcgat ttcaggagga aataaaagaa aacacaaaga acgacaaaca aatggtccaa     300

tatatttaca aatacacaag ttatcctgac cctatattgt tgatgaaaag tgctagaaat     360

agttgttggt ctaaagatgc agaatatgga ctctattcca tctatcaagg gggaatattt     420

gagcttaagg aaaatgacag aatttttgtt tctgtaacaa atgagcactt gatagacatg     480

gaccatgaag ccagtttttt bggggccttt ttagttggct aactgacctg gaaagaaaaa     540

gcaataacct caaagtgact attcagtttt caggatgata cactatgaag atgtttcaaa     600

aaatctgacc aaaacaaaca aacagaaaac agaaaacaaa aaaacctcta tgcaatctga     660

gtagagcagc cacaaccaaa aaattctaca acacacactg ttctgaaagt gactcactta     720

tcccaagaga atgaaattgc tgaaagatct ttcaggactc tacctcatat cagtttgcta     780

gcagaaatct agaagactgt cagcttccaa acattaatgc aatggttaac atcttctgtc     840

tttataatct actccttgta aagactgtag aagaaagagc aacaatccat ctctcaagta     900

gtgtatcaca gtagtagcct ccaggtttcc ttaaggacaa acatccttaa gtcaaaagag     960

agaagaggca ccactaaaag atcgcagttt gcctggtgca g                        1001


<210>  3
<211>  20
<212>  DNA
<213>  artificial sequence

<220>
<223>  primer

<400>  3
acaacataca gctgggccag                                                  20

<210>  4
<211>  20
<212>  DNA
<213>  artificial sequence

<220>
<223>  primer

<400>  4
gtagtcgttg gaaaggaggg                                                   20


<210>  5
<211>  20
<212>  DNA
<213>  artificial sequence

<220>
<223>  primer

<400>  5
aaacaggcct tgtgcctatg                                                   20


<210>  6
<211>  22
<212>  DNA
<213>  artificial sequence

<220>
<223>  primer

<400>  6
gctttcatga agagttgcaa tg                                                22


<210>  7
<211>  20
<212>  DNA
<213>  artificial sequence

<220>
<223>  primer

<400>  7
tggcagaact ggaagagacc                                                   20


<210>  8
<211>  20
<212>  DNA
<213>  artificial sequence

<220>
<223>  primer

<400>  8
catcagcaat gtgggaagaa                                                   20


<210>  9
<211>  20
<212>  DNA

<213>  artificial sequence

<220>
<223>  primer

<400>  9
ccacatttgg ctgacatcac                                                    20


<210>  10
<211>  20
<212>  DNA
<213>  artificial sequence

<220>
<223>  primer

<400>  10
atgatggagg aggaggcttt                                                    20


<210>  11
<211>  20
<212>  DNA
<213>  artificial sequence

<220>
<223>  primer

<400>  11
gtttctcttt ggaccctacc                                                    20


<210>  12
<211>  21
<212>  DNA
<213>  artificial sequence

<220>
<223>  primer

<400>  12
tttcctgagg ccagttatgt c                                                  21


<210>  13
<211>  20
<212>  DNA
<213>  artificial sequence

<220>
<223>  primer

<400>  13
ctcccaacag ttcccaatgt                                                    20


<210>  14
<211>  20
<212>  DNA
<213>  artificial sequence

<220>

<223>  primer

<400>  14
ccttaaggaa acctggaggc                                                   20


<210>  15
<211>  20
<212>  DNA
<213>  artificial sequence

<220>
<223>  primer

<400>  15
ggggcctttt tagttggcta                                                   20


<210>  16
<211>  20
<212>  DNA
<213>  artificial sequence

<220>
<223>  primer

<400>  16
cagcacaact caacccagaa                                                   20


<210>  17
<211>  20
<212>  DNA
<213>  artificial sequence

<220>
<223>  primer

<400>  17
tcctgggaat catcaaggag                                                   20


<210>  18
<211>  20
<212>  DNA
<213>  artificial sequence

<220>
<223>  primer

<400>  18
gcaggaacct cccaatttct                                                   20


<210>  19
<211>  20
<212>  DNA
<213>  artificial sequence

<220>
<223>  primer

<400>  19

aatctgagta gagcagccac                                                    20

<210>  20
<211>  20
<212>  DNA
<213>  artificial sequence

<220>
<223>  primer

<400>  20
ccacagagaa aggaagcagg                                                    20

<210>  21
<211>  20
<212>  DNA
<213>  artificial sequence

<220>
<223>  primer

<400>  21
ttaccctgtg tgtgcctcag                                                    20

<210>  22
<211>  20
<212>  DNA
<213>  artificial sequence

<220>
<223>  primer

<400>  22
tgagcttcct agctgccaat                                                    20

<210>  23
<211>  21
<212>  DNA
<213>  artificial sequence

<220>
<223>  primer

<400>  23
cctcctgaaa tcgaaagtat g                                                  21

<210>  24
<211>  20
<212>  DNA
<213>  artificial sequence

<220>
<223>  primer

<400>  24
tggcatgatc tcaccacact                                                    20

## Description

### FIELD OF THE INVENTION

The present invention relates to the field of the medicine. In particular, it relates to methods for evaluating the radio-sensitivity and the risk of adverse effects for an individual.

### BACKGROUND OF THE INVENTION

Inter-individual differences in radio-sensitivity are extensively documented since the discovery of radiation, and are causally related to the varying levels of toxicity that all radiotherapy patients will experience. However, except for very rare monogenic diseases such as homozygous carriers of a mutated *ATM* gene, the genetic basis of individual radio-sensitivity is poorly characterized. Nevertheless, numerous studies have indicated a correlation between cellular- and clinical radio-sensitivity. This is exemplified by the association between low CD8+-lymphocyte apoptosis and radiation induced late toxicity (Ozsahin et al., 2005, Clin Cancer Res. 11:7426-7433). However, no consensus exists on available biological tests that can be reliably used for prediction of early- and/or late clinical adverse effects associated with radiotherapy (Finnon et al., 2012, Radiother Oncol. 105:329-336; Greve et al., 2011, PLoS ONE. 7:e47185-e47185).

Dose limitation due to side-effects observed in a minority of patients greatly reduces efficacy of radiotherapy, because of the direct relationship between radiation dose and tumor control. Beyond technical developments such as optimizing radiation delivery, improving radiotherapy outcome requires a better understanding of the underlying mechanisms of individual radiosensitivity (West and Barnett, 2011, Genome Med. 3:52) that will ultimately allow individualized radiotherapy.

Therefore, methods allowing to foreseeing radiosensitivity would be useful for the clinicians in order to optimize the cancer therapy.

### SUMMARY OF THE INVENTION

The present invention provides a method for assessing the individual radiosensitivity associated to apoptosis susceptibility of lymphocytes and risk of developing complications in irradiated normal tissues. The method comprises genotyping particular polymorphisms in TNFSF10/TRAIL gene.

The present invention relates to the use of a single nucleotide polymorphism (SNP) selected from the group consisting of rs1131532, rs3815496 and any SNP having a linkage disequilibrium with an absolute value for r² of 0.85 therewith as a biomarker for assessing the risk of developing adverse effects after radiotherapy in a subject, wherein said adverse effects are selected for the group consisting of acute and/or subacute dermatitis and lymphocyte apoptosis.

The present invention also relates to a method for assessing the risk of developing adverse effects after radiotherapy in a subject, wherein the method comprises determining the allele of a single nucleotide polymorphism (SNP) selected from the group consisting of rs1131532, rs3815496 and any SNP having a linkage disequilibrium with an absolute value for r² of 0.85 therewith in a subject sample; wherein said adverse effects are acute and/or subacute dermatitis and/or lymphocyte apoptosis. Preferably, the step of determining the allele is a step of genotyping by sequencing, selective hybridization and/or selective amplification, enzyme-based methods or methods relying on differences in the conformation, weight or size of the molecules. Preferably, the subject sample is saliva, urine, whole blood, plasma, or serum sample. More preferably the subject is human.

The present invention also relates to a method for determining the radiation dose suitable for a subject, comprising performing the method for assessing the risk of developing adverse effects after radiotherapy in a subject as disclosed above, and selecting the suitable radiation dose for the subject so as a reduced radiation dose is selected if the subject has at least one allele of the SNP associated with an increased risk of developing adverse effects after radiotherapy and a maximal radiation dose is selected if the subject does not present any allele of the SNP associated with an increased risk of developing adverse effects after radiotherapy.

The present invention further relates to the use of a kit for assessing the risk of developing adverse effects after radiotherapy in a subject, said adverse effects being acute and/or subacute dermatitis and/or lymphocyte apoptosis, or for determining the radiation dose and/or selecting the radiotherapy delivery suitable for a subject, wherein the kit comprises at least one nucleic acid probe capable of specifically binding or hybridizing a SNP according to the invention, and/or at least one set of primers suitable for use in amplification reaction suitable for amplifying a SNP as defined in the present invention.

Preferably, the SNP having a linkage disequilibrium with rs3815496 is selected in one of the following groups consisting of
a) rs1131532, rs9811673, rs9811639, rs3136600, rs3815496, rs3181143, rs3181142, rs3136606, rs3136607, rs3819773, rs1126161, rs63562861, rs1131542, rs1131568, rs11720451, rs9859809, rs6807069, rs6783667, rs9869255, rs9811650, rs9811646, rs62282578, rs1992868, rs1992869, rs6414542, rs1597086, rs9880799, and rs4894557, more preferably from the group consisting of rs3815496, rs3181143, rs3181142, rs3136606, rs3136607, rs3819773, rs1126161, rs63562861, rs1131542, rs1131568, rs11720451, rs9859809, rs6807069, rs6783667, rs9869255, rs9811650, rs9811646, rs62282578, rs1992868, rs1992869, rs6414542, rs1597086, rs9880799, and rs4894557;
b) rs1131532, rs1131542, rs3815496, rs3181143, rs3136600, rs3136606, rs3136607, rs3819773, rs1126161, rs63562861, rs11720451, rs9859809, rs6807069, rs6783667, rs9811673, rs9869255, rs9811650, rs9811646, rs62282578, rs1992868, rs1597086, rs9811639, rs1992869, rs6414542, rs9880799, rs4894557, rs9859259, and rs9880475, more preferably from the group consisting of rs1131532, rs3815496, rs3181143, rs3136600, rs3136606, rs3136607, rs3819773, rs1126161, rs63562861, rs1131542, rs11720451, rs9859809, rs6807069, rs6783667, rs9811673, rs9869255, rs9811650, rs9811646, rs62282578, rs1992868, and rs1597086;
c) rs3136600;
d) rs1131532, rs9811673, rs9811639, rs3136600, rs3815496, rs3181143, rs3136606, rs3136607, rs3819773, rs1126161, rs63562861, rs1131542, rs11720451, rs9859809, rs6807069, rs6783667, rs9869255, rs9811650, rs9811646, rs62282578, rs1992868, rs1992869, rs6414542, rs1597086, rs9880799 and rs4894557.

Alternatively, the SNP having a linkage disequilibrium with rs1131532 is selected in one of the following groups consisting of
a) rs3815496, rs1131542, rs63562861, rs1131568, rs11720451, rs9859809, rs3819773, rs3136607, rs3136606, rs6807069, rs6783667, rs9869255, rs9811650, rs9811646, rs62282578, rs3181143, rs3181142, rs1992868, rs1992869, rs6414542, rs1597086, rs9880799, rs1126161, rs4894557, rs9811673 and rs9811639;
b) rs3815496, rs1131542, rs63562861, rs11720451, rs9859809, rs3819773, rs3136607, rs3136606, rs3136600, rs6807069, rs6783667, rs9811673, rs9869255, rs9811650, rs9811646, rs62282578, rs3181143, rs1992868, rs1597086, rs1126161, rs9811639, rs1992869, rs6414542, rs9880799, rs4894557, rs9859259 and rs9880475, more preferably from the group consisting of rs3815496, rs1131542, rs63562861, rs11720451, rs9859809, rs3819773, rs3136607, rs3136606, rs3136600, rs6807069, rs6783667, rs9811673, rs9869255, rs9811650, rs9811646, rs62282578, rs3181143, rs1992868, rs1597086 and rs1126161
c) rs1131542, rs11720451, rs3819773, rs3136607, rs3136606, rs6807069, rs6783667, rs12696330, rs9811673, rs9869255, rs9811650, rs9811646, rs9811639, rs62282578, rs9849142, rs1992868, rs1992869, rs6414542, rs1597086, rs9880799, rs9859809 and rs63562861;
d) rs3815496, rs1131542, rs63562861, rs11720451, rs9859809, rs3819773, rs3136607, rs3136606, rs6807069, rs6783667, rs9869255, rs9811650, rs9811646, rs62282578, rs3181143, rs1992868, rs1992869, rs6414542, rs1597086, rs9880799, rs1126161, rs4894557, rs9811673, and rs9811639.

In a preferred embodiment, the SNP having a linkage disequilibrium with rs1131532 and rs3815496 is selected from the group consisting of rs9811673, rs9811639, rs3815496, rs3181143, rs3136606, rs3136607, rs3819773, rs1126161, rs63562861, rs1131542, rs11720451, rs9859809, rs6807069, rs6783667, rs9869255, rs9811650, rs9811646, rs62282578, rs1992868, rs1992869, rs6414542, rs1597086, rs9880799 and rs4894557.

In a most preferred embodiment, the SNP is rs3815496 or rs1131532.

### BRIEF DESCRIPTION OF THE FIGURES

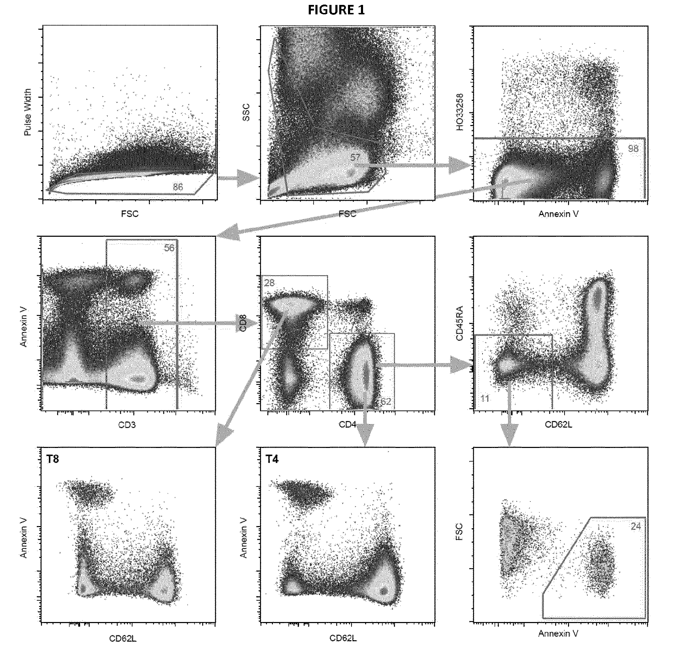

**Figure 1**. Figure 1 shows the gating strategy leading to the quantification of annexinV positive cells in T4EM, 18 hours after irradiation at 2Gy of a PBMC sample, dot plots show absence of AnnexinV positive cells in T4 and T8 CD62L lymphocytes and AnnexinV positive cells in T4EM lymphocytes.

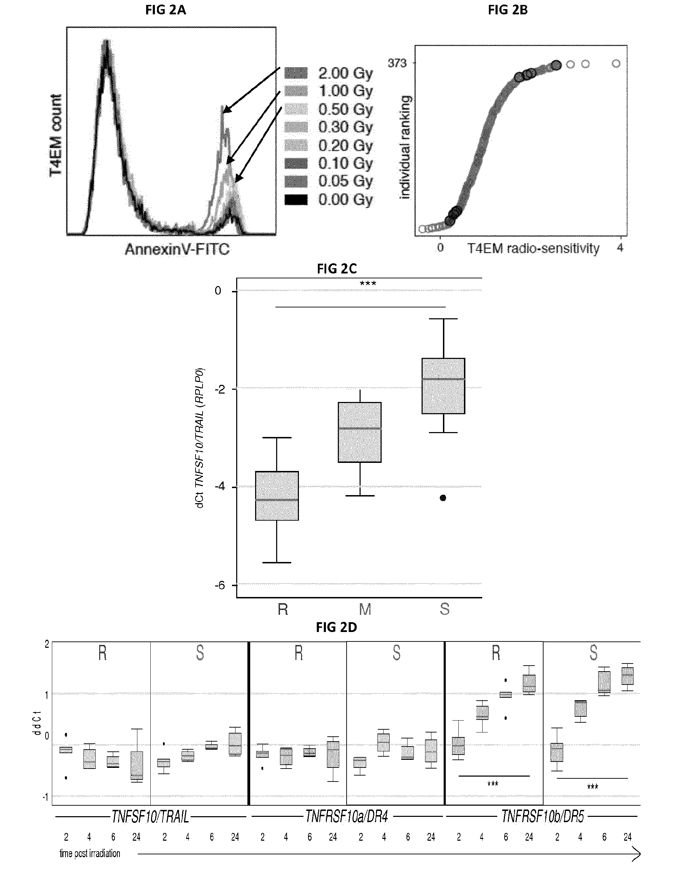

**Figure 2****. *TRAIL*/*TNFSF10* mRNA level correlates with T4EM cellular radiosensitivity phenotype.** (FIG 2A) Overlaid histograms of AnnexinV fluorescence intensity of T4EM lymphocytes 18 hours after irradiation at the indicated doses. (FIG 2B) Ranking of 373 individuals according to their T4EM lymphocytes radiosensitivity measured by the level of apoptosis (grey circles). T4EM lymphocytes from four "sensitive" and four "resistant" unrelated individuals (black circles) were selected for micro-array expression analysis. T4EM lymphocytes from fifteen "sensitive" , "median" and "resistant" unrelated individuals were used for qPCR. (FIG 2C) Quantitative PCR analysis of expression level of *TRAIL*/*TNFSF10* in sorted T4EM lymphocytes from 15 "resistant" (R), 15 "median" (M), and 15 "sensitive" unrelated individuals (S). Results are presented as box plots of dCt with respect to the internal reference gene (*RPLP0*) with median values (red) (****p*<10⁻³). (FIG 2D) Multiplexed qPCR analysis of *TNFSF10*/*TRAIL* (left panels), *TNFRSF10a*/*DR4* (middle panels) and *TNFRSF10b*/*DR5* (right panels) in sorted T4EM lymphocytes from 5 "resistant" (R) and 5 "sensitive" (S) unrelated individuals, 2, 4, 6, and 24 hours after irradiation at 2 Gy. Results are presented as the difference in dCt (ddCt) between non-irradiated sample and the dCt of the test sample, for each timepoint post-irradiation (hours), and are presented as box plots with median line (red) (****p*<10⁻³).

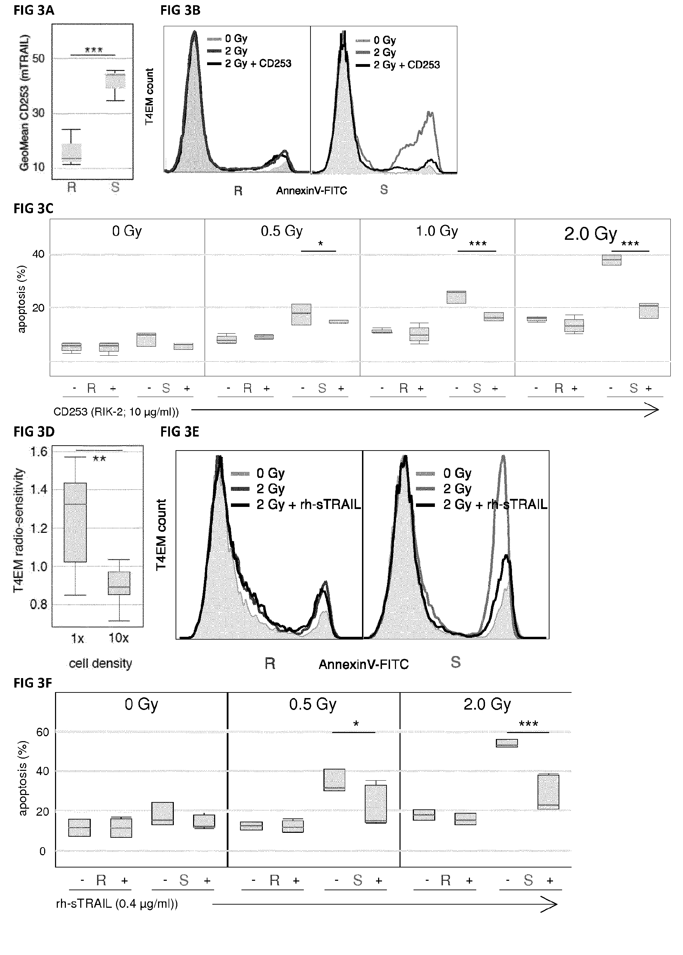

**Figure 3****. Membrane bound TRAIL (mTRAIL) mediates pro-apoptotic autocrine signaling in irradiated T4EM lymphocytes**. (FIG 3A) mTRAIL level by flow cytometric analysis of resting T4EM lymphocytes from 4 "sensitive" (S) and 4 "resistant" (R) samples from unrelated individuals. Box plot representation with median line (****p*<10⁻³). (FIG 3B) Overlaid histograms of AnnexinV fluorescence intensity of "resistant" (left panel) or "sensitive" (right panel) samples after 18 hours without irradiation (shaded grey), after 2 Gy irradiation in the absence ("resistant" / "sensitive") or presence (black) of TRAIL blocking antibody. (FIG 3C) Percentage of apoptotic T4EM lymphocytes from 4 "sensitive" (S) and 4 "resistant" (R) samples, 18 hours after irradiation at 0, 0.5, 1, and 2 Gy, in the absence (-) or presence (+) of TRAIL blocking antibody. Box plot representation with median line (red) (*p<0.05, ****p*<10⁻³). (FIG 3D) Radiosensitivity of 9 samples from unrelated "sensitive" individuals after 2 Gy irradiation at cell concentrations of 10⁵ /ml (1X) and 10⁶ /ml (10X) (***p*<10⁻²). (FIG 3E) Overlaid histograms of AnnexinV fluorescence intensity of "resistant" (left panel) or "sensitive" (right panel) samples after 18 hours without irradiation (shaded grey), after a 2 Gy irradiation in the absence ("resistant" / "sensitive") or presence of 0.4 µg/ml rh-sTRAIL (black). (FIG 3F) Percentage of apoptotic T4EM lymphocytes from 4 "sensitive" (S) and 4 "resistant" (R) samples, 18 hours after irradiation at 0, 0.5, 1, and 2 Gy, in the absence (-) or presence (+) of rh-sTRAIL (0.4 µg/ml). Box plot representation with median line (**p*<0.05, ****p*<10⁻³).

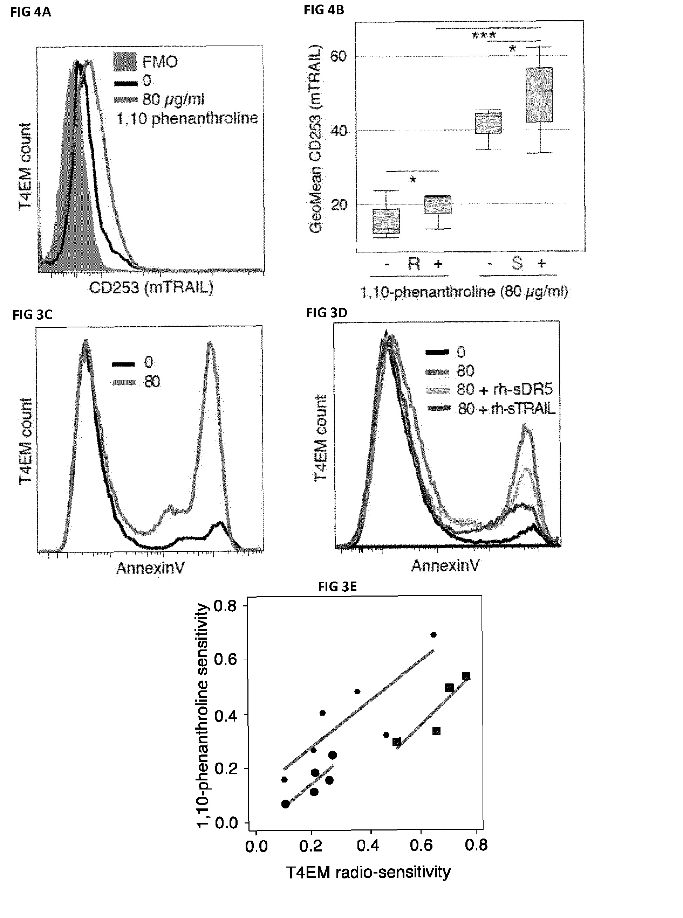

**Figure 4****. Matrix metalloprotease inhibitor 1,10-phenanthroline induces T4EM lymphocytes apoptosis that correlates with T4EM lymphocyte radio- sensitivity.** (FIG 4A) mTRAIL level by flow cytometric analysis on T4EM lymphocytes after 2 hours of culture without (black) and with 80 µg/ml 1,10-phenanthroline. Corresponding FMO control histogram is shaded grey. (FIG 4B) Effect of treatment with 80 µg/ml of 1,10-phenanthroline on mTRAIL expression on T4EM lymphocytes from 4 "sensitive" (S) and 4 "resistant" (R) samples. Box plot representation with median line (***p<0.05 and ****p*<10⁻³). (FIG 4C) Overlaid histograms of AnnexinV fluorescence intensity of T4EM lymphocytes after two hours without (black) and with 80 µg/ml 1,10-phenanthroline. (D) Overlaid histograms of AnnexinV fluorescence intensity of T4EM lymphocytes after 2 hours without (black) and with 80 µg/ml 1,10-phenanthroline in the absence or in the presence of 4 µg/ml rh-sTRAIL or 4 µg/ml rh-sDR5. (E) Scatter plot of T4EM lymphocyte radiosensitivity (abscissa) versus apoptosis induced by 1,10-phenanthroline (ordinate). Three independent experiments comprising 4 (cryopreserved; ▪), 5 (fresh;●), and 6 (cryopreserved; ▲) samples. Regression lines were calculated per experiment.

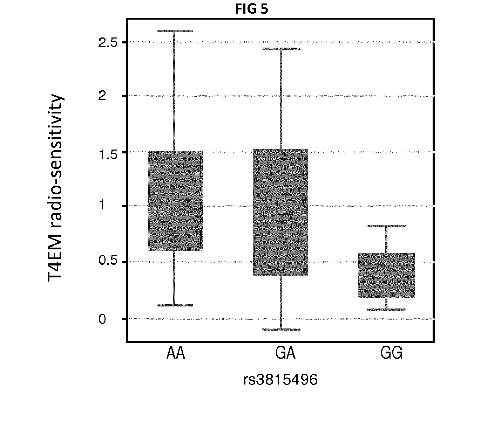

**Figure 5****. Association between *TRAIL*/*TNFSF10* SNPs, radiosensitivity and radio-induced acute and subacute dermatitis.** Box plot representation with median line (red) of 126 unrelated individuals by genotype at rs3815496, counting 65 AA, 55 GA and 6 GG.

### DETAILED DESCRIPTION OF THE INVENTION

The inventors identify SNPs located in the TRAIL/TNFSF10 gene which can be used for predicting the radio-sensitivity of a subject. In particular, the identified SNP are associated with higher risk or probability of acute and/or subacute dermatitis, and/or lymphocyte apoptosis. The identified SNPs are rs3815496 and rs1131532 which present a linkage disequilibrium between each other.

In a first aspect, the identified SNPs and any SNP having a linkage disequilibrium with an absolute value for r² of at least 0.85 can be used to determine radio-sensitivity of a subject after a radiation exposure, especially an accidental radiation exposure for instance due to ingestion, inhalation or deposition of radioactive materials. Then, if the subject is determined to present radio-sensitivity, he can benefit of an increased medical monitoring or receive an appropriate treatment such as preventive treatment.

However, the main source of radiation exposure is the radiotherapy, in particular for cancer treatment. Therefore, the main aspect of the present invention is the use of the identified SNPs and any SNP having a linkage disequilibrium with an absolute value for r² of at least 0.85 as a biomarker for assessing the risk of developing adverse effects after radiotherapy in a subject. In particular, the adverse effects are selected for the group consisting of acute and/or subacute dermatitis and lymphocyte apoptosis, especially of CD4+ effector memory lymphocytes.

The term "radiotherapy" is commonly used in the art to refer to multiple types of radiation therapy including internal and external radiation therapy, radioimmunotherapy, and the use of various types of radiation including X-rays, gamma rays, alpha particles, beta particles, photons, electrons, neutrons, radioisotopes, and other forms of ionizing radiation. Preferably, the radiotherapy involves the use of X-rays or gamma-rays.

The term "TRAIL/TNFSF10 gene" as used herein, refers to a polynucleotide encoding a Tumor necrosis factor (ligand) superfamily, member 10 polypeptide (Gene ID: 8743). An exemplary sequence for this protein is Genbank Accession Number: NP_003801.1. An exemplary sequence for the polynucleotide (mRNA) encoding this precursor is Genbank Accession Number: NM_003810.3.

The methods of the invention as disclosed herein may be *in vivo, ex vivo* or *in vitro* methods, preferably *in vitro* methods.

The present invention further relates to a method for assessing the risk of developing adverse effects after radiotherapy in a subject, wherein the method comprises determining the allele of a single nucleotide polymorphism (SNP) selected from the group consisting of rs1131532, rs3815496 and any SNP having a linkage disequilibrium with an absolute value for r² of 0.85 therewith in a subject sample. Preferably, said adverse effects are acute and/or subacute dermatitis, and/or lymphocyte apoptosis.

In a particular aspect, the presence of at least one allele A of SNP rs3815496 is indicative of a risk of developing adverse effects after radiotherapy or a predisposition to adverse effects after radiotherapy. The a risk of developing adverse effects after radiotherapy is higher in a subject carrying one or two alleles A of SNP rs3815496 compared to a subject only carrying alleles G. The risk or predisposition is increased in a subject who is a homozygous carrier of the allele A in comparison to a heterozygous carrier.

In another particular aspect, the presence of at least one allele C of SNP rs1131532 is indicative of a risk of developing adverse effects after radiotherapy or a predisposition to adverse effects after radiotherapy. The a risk of developing adverse effects after radiotherapy is higher in a subject carrying one or two alleles C of SNP rs1131532 compared to a subject only carrying alleles G. The risk or predisposition is increased in a subject who is a homozygous carrier of the allele C in comparison to a heterozygous carrier.

The present invention further relates to a method for optimizing the treatment to a subject, the method comprising performing the method for assessing the risk of developing adverse effects after radiotherapy in a subject as detailed above.

If the subject has at least one allele of the SNP associated with an increased risk of developing adverse effects after radiotherapy, he can benefit of an increased medical monitoring or receive an appropriate treatment such as preventive treatment. For instance, the preventive treatment can include a treatment with recombinant TRAIL or TRAIL agonistic antibodies. This treatment could be provided to the subject before the radiotherapy.

Depending on the risk of developing adverse effects after radiotherapy for a subject, the physician can optimize the radiotherapy procedure to the subject, for instance by selecting the most appropriate delivery means and/or selecting the radiation dose.

In a particular embodiment, the method is suitable for determining the radiation dose suitable for a subject. The method comprises performing the method for assessing the risk of developing adverse effects after radiotherapy in a subject as detailed above and selecting the suitable radiation dose for the subject so as a reduced radiation dose is selected if the subject has at least one allele of the SNP associated with an increased risk of developing adverse effects after radiotherapy and a maximal radiation dose is selected if the subject does not present any allele of the SNP associated with an increased risk of developing adverse effects after radiotherapy. The method may further comprises a step of administering/applying the selected radiation dose to the subject. In a preferred embodiment, the selected radiation dose is administered by conformational irradiation. Indeed, this technology is suitable for the delivery of a precise radiation dose to the subject at a precise location.

The inventors identified two SNPs, namely rs3815496 and rs1131532.
rs3815496 (SEQ ID No 1) 
CCTCCTGGAT TCACACCATT CTCCTGCCTC AGCCTCCTGA GTAGTTGGGA CTATAGGTGC
CTGCCACCAT GCCCAGCTAA TTTTTTTGTA TTTTTAGTAG AGATGGGGTT TCACCGTGTT
 
AGCCAGGATA GTCTCAATCT CCTGACCATC TGTTTGTTGG TACCACTCTG TTTATTTCTG
TATGTAATTC AAGGTGGAAA TTTGCCAATA CGTAGTTATT TGCACAATTT AACTCAGCAG
GATTATTACA CAGCTACAGC TATTTCTTTA TTTATATTTT TGGAAAAGGG ATAGAATCTT
CACTTTGTTG TATCTTCACT TTGTTTCTCT TTGGACCCTA CCCGTGAGAC ACTAATAAAT
TATAATCCAT GTTACTGCTT ATTCTTTCCG TGTAAAGTAC TTTTCAAGTT GATCATAAGC
TGAGGTAGGT TGATCATAAT ATCAAAATGT TATTTTTATG AAAACTCATA GAATCAACTT
TGGAAGCCCA GTATGCTTAC
R
ATGGCTGATT GACAACACCT ATGAAGAGCA AGGTATTGGA GAACTAGTTT GGCAAGTGTT
GATGACCCTT TATCATTTCT CTTTTAGAAA AGCAACAAAA TATTTCTCCC CTAGTGAGAG
AAAGAGGTCC TCAGAGAGTA GCAGCTCACA TAACTGGGAC CAGAGGAAGA AGCAACACAT
TGTCTTCTCC AAGTAAGAGA AACAACAAAT AAGTGACTTA AGGGAAAATA ATGGAAGGAT
TGTGCCAAGT AACTTATTTT TTAAAGAATT TTATCTGAAA TGTTTATATG GCTATCTACA
AAATGCTCAG TATTAATCAG ATCTCAGATT GACTGTTTTC AGATGCAAGA TTACAGACAA
ACAGCTCAAT TATTGCCACG ACATAACTGG CCTCAGGAAA ATTACAAAGT GGGAGAATTT
TGTCTTTGCA GTTTTCAGGA TCCCATTTTT TTTCCTTCTT TAGAGTAATT TCTCGCTCTT
TCTTTCTTTC TTTTTCTTTC
 with R being A or G.
rs1131532 (SEQ ID No 2) 
ACAAAAATAT AGGCTGGCCA CAGCTGCAAA AAAGCTCCTT GCTTTGCTAC AAATTCCCTT
CTGTTAACGC TCTTTCTCTC TAAATTTCCT ACAGACTCCA AGAATGAAAA GGCTCTGGGC
CGCAAAATAA ACTCCTGGGA ATCATCAAGG AGTGGGCATT CATTCCTGAG CAACTTGCAC
TTGAGGAATG GTGAACTGGT CATCCATGAA AAAGGGTTTT ACTACATCTA TTCCCAAACA
TACTTTCGAT TTCAGGAGGA AATAAAAGAA AACACAAAGA ACGACAAACA AATGGTCCAA
TATATTTACA AATACACAAG TTATCCTGAC CCTATATTGT TGATGAAAAG TGCTAGAAAT
AGTTGTTGGT CTAAAGATGC AGAATATGGA CTCTATTCCA TCTATCAAGG GGGAATATTT
GAGCTTAAGG AAAATGACAG AATTTTTGTT TCTGTAACAA ATGAGCACTT GATAGACATG
GACCATGAAG CCAGTTTTTT
B
GGGGCCTTTT TAGTTGGCTA ACTGACCTGG AAAGAAAAAG CAATAACCTC AAAGTGACTA
TTCAGTTTTC AGGATGATAC ACTATGAAGA TGTTTCAAAA AATCTGACCA AAACAAACAA
ACAGAAAACA GAAAACAAAA AAACCTCTAT GCAATCTGAG TAGAGCAGCC ACAACCAAAA
AATTCTACAA CACACACTGT TCTGAAAGTG ACTCACTTAT CCCAAGAGAA TGAAATTGCT
GAAAGATCTT TCAGGACTCT ACCTCATATC AGTTTGCTAG CAGAAATCTA GAAGACTGTC
AGCTTCCAAA CATTAATGCA ATGGTTAACA TCTTCTGTCT TTATAATCTA CTCCTTGTAA
AGACTGTAGA AGAAAGAGCA ACAATCCATC TCTCAAGTAG TGTATCACAG TAGTAGCCTC
CAGGTTTCCT TAAGGGACAA CATCCTTAAG TCAAAAGAGA GAAGAGGCAC CACTAAAAGA
TCGCAGTTTG CCTGGTGCAG
 with B being C, G or T.

rs3815496 and rs1131532 present a linkage disequilibrium with an absolute value for r² of 0.895 in population European super population and 1 in African super population.

A "polymorphic site" or "polymorphism site" or "polymorphism" or "single nucleotide polymorphism site" (SNP site) or "single nucleotide polymorphism" (SNP) as used herein is the locus or position with a given sequence at which divergence occurs. A "polymorphism" is the occurrence of two or more forms of a gene or position within a gene (allele), in a population. Polymorphic sites have at least two alleles, each occurring at a frequency of greater than 1 percent, and may be greater than 10 percent or 20 percent of a selected population. Polymorphic sites may be at known positions within a nucleic acid sequence or may be determined to exist. Polymorphisms may occur in both the coding regions and the noncoding regions (for example, promoters, introns or untranslated regions) of genes. Polymorphisms may occur at a single nucleotide site (SNP) or may involve an insertion or deletion as described herein. The SNPs are identified by their refSNP of dbSNP database of NCBI (http://www.ncbi.nlm.nih.gov/SNP).

As used herein "linkage disequilibrium" (LD) is the occurrence in a population of certain combinations of linked alleles in greater proportion than expected from the allele frequencies at the loci. Accordingly, if the genotype of a first locus is in LD with a second locus (or third locus etc.), the determination of the allele at only one locus would necessarily provide the identity of the allele at the other locus. Accordingly, two SNPs that have a high degree of LD may be equally useful in determining the identity of the allele of interest. Therefore, knowing the identity of the allele at one SNP is representative of the allele identity at another SNP in LD. Accordingly, the determination of the genotype of a single locus can provide the identity of the genotype of any locus in LD therewith and the higher the degree of linkage disequilibrium the more likely that two SNPs may be used interchangeably. When evaluating loci for LD those sites within a given population having a high degree of linkage disequilibrium (i.e. an absolute value for r² is 0.5) are potentially useful in predicting the identity of an allele of interest (i.e. associated with the radio-sensitivity or the adverse effects). A high degree of linkage disequilibrium may be represented by an absolute value for r² > 0.7 or by an absolute value for r > 0.8. Additionally, a high degree of linkage disequilibrium may be represented by an absolute value for r² > 0.85 or by an absolute value for r² > 0.9 or by an absolute value for r² > 0.95. In a preferred embodiment, a high degree of linkage disequilibrium is represented by an absolute value for r² of 1. More particularly, when the absolute value for r² is 1, two SNPs are interchangeable.

LD may be useful for genotype-phenotype association studies. For example, if a specific allele at one SNP site (e.g. "A") is the cause of a specific clinical outcome (e.g. call this clinical outcome "B") in a genetic association study then, by mathematical inference, any SNP (e.g. "C") which is in significant LD with the first SNP, will show association with the clinical outcome. That is, if A is associated (^{∼}) with B, i.e. A-B and C-A then it follows that C-B.

Tools are available for determining the SNP is linkage disequilibrium with another SNP. In particular, such data are available on the website www.broadinstitute.org/mpg/snap/.

The SNPs having a linkage disequilibrium with an absolute value for r² of at least 0.85 with rs3815496 can be selected in one of the following groups consisting of
1. rs1131532, rs9811673, rs9811639, rs3136600, rs3815496, rs3181143, rs3181142, rs3136606, rs3136607, rs3819773, rs1126161, rs63562861, rs1131542, rs1131568, rs11720451, rs9859809, rs6807069, rs6783667, rs9869255, rs9811650, rs9811646, rs62282578, rs1992868, rs1992869, rs6414542, rs1597086, rs9880799, and rs4894557, more preferably from the group consisting of rs3815496, rs3181143, rs3181142, rs3136606, rs3136607, rs3819773, rs1126161, rs63562861, rs1131542, rs1131568, rs11720451, rs9859809, rs6807069, rs6783667, rs9869255, rs9811650, rs9811646, rs62282578, rs1992868, rs1992869, rs6414542, rs1597086, rs9880799, and rs4894557;
2. rs1131532, rs1131542, rs3815496, rs3181143, rs3136600, rs3136606, rs3136607, rs3819773, rs1126161, rs63562861, rs11720451, rs9859809, rs6807069, rs6783667, rs9811673, rs9869255, rs9811650, rs9811646, rs62282578, rs1992868, rs1597086, rs9811639, rs1992869, rs6414542, rs9880799, rs4894557, rs9859259, and rs9880475, more preferably from the group consisting of rs1131532, rs3815496, rs3181143, rs3136600, rs3136606, rs3136607, rs3819773, rs1126161, rs63562861, rs1131542, rs11720451, rs9859809, rs6807069, rs6783667, rs9811673, rs9869255, rs9811650, rs9811646, rs62282578, rs1992868, and rs1597086;
3. rs3136600;
4. rs1131532, rs9811673, rs9811639, rs3136600, rs3815496, rs3181143, rs3136606, rs3136607, rs3819773, rs1126161, rs63562861, rs1131542, rs11720451, rs9859809, rs6807069, rs6783667, rs9869255, rs9811650, rs9811646, rs62282578, rs1992868, rs1992869, rs6414542, rs1597086, rs9880799 and rs4894557.

The SNPs having a linkage disequilibrium with an absolute value for r² of at least 0.85 with rs1131532 can be selected in one of the following groups consisting of
1- rs3815496, rs1131542, rs63562861, rs1131568, rs11720451, rs9859809, rs3819773, rs3136607, rs3136606, rs6807069, rs6783667, rs9869255, rs9811650, rs9811646, rs62282578, rs3181143, rs3181142, rs1992868, rs1992869, rs6414542, rs1597086, rs9880799, rs1126161, rs4894557, rs9811673 and rs9811639;
2- rs3815496, rs1131542, rs63562861, rs11720451, rs9859809, rs3819773, rs3136607, rs3136606, rs3136600, rs6807069, rs6783667, rs9811673, rs9869255, rs9811650, rs9811646, rs62282578, rs3181143, rs1992868, rs1597086, rs1126161, rs9811639, rs1992869, rs6414542, rs9880799, rs4894557, rs9859259 and rs9880475, more preferably from the group consisting of rs3815496, rs1131542, rs63562861, rs11720451, rs9859809, rs3819773, rs3136607, rs3136606, rs3136600, rs6807069, rs6783667, rs9811673, rs9869255, rs9811650, rs9811646, rs62282578, rs3181143, rs1992868, rs1597086 and rs1126161
3- rs1131542, rs11720451, rs3819773, rs3136607, rs3136606, rs6807069, rs6783667, rs12696330, rs9811673, rs9869255, rs9811650, rs9811646, rs9811639, rs62282578, rs9849142, rs1992868, rs1992869, rs6414542, rs1597086, rs9880799, rs9859809 and rs63562861;
4- rs3815496, rs1131542, rs63562861, rs11720451, rs9859809, rs3819773, rs3136607, rs3136606, rs6807069, rs6783667, rs9869255, rs9811650, rs9811646, rs62282578, rs3181143, rs1992868, rs1992869, rs6414542, rs1597086, rs9880799, rs1126161, rs4894557, rs9811673, and rs9811639.

More particularly, item 1 relates to the European super population, item 2 to the African super population, item 3 to the East Asian super population and item 4 relates to the SNPs in common between European and African super populations.

In a preferred embodiment, the SNPs can be selected in the group consisting of rs9811673, rs9811639, rs3815496, rs3181143, rs3136606, rs3136607, rs3819773, rs1126161, rs63562861, rs1131542, rs11720451, rs9859809, rs6807069, rs6783667, rs9869255, rs9811650, rs9811646, rs62282578, rs1992868, rs1992869, rs6414542, rs1597086, rs9880799 and rs4894557. They have a linkage disequilibrium with an absolute value for r² of at least 0.85 with both rs1131532 and rs3815496 and being in common between European and African super populations.

The methods, uses and kits according to the invention can be performed with one SNP as disclosed herein or with a combination thereof.

In a preferred embodiment, the subject is a human being. Optionally, the subject can belong to European super population, African super population or East Asian super population.

### Genotyping

The method comprises determining the alleles of a SNP of the invention in a sample from the subject. It may comprise a previous step of providing a sample from the subject.

The term "sample", as used herein, refers to a composition that is obtained or derived from a subject of interest that contains a cell or genetic materials suitable for genotyping a SNP. The source of the sample may be solid tissue as from a fresh, frozen and/or preserved organ or tissue sample or biopsy or aspirate; blood or any blood constituents; bodily fluids such as cerebral spinal fluid, amniotic fluid, peritoneal fluid, or interstitial fluid. The sample may also be primary or cultured cells. Optionally, the sample can be obtained from a disease tissue/organ, especially from a cancer or tumor. The tissue sample may contain compounds which are not naturally intermixed with the tissue in nature such as preservatives, anticoagulants, buffers, fixatives, nutrients, antibiotics, or the like. In a preferred embodiment, the sample is saliva, urine, whole blood, plasma, or serum sample. The sample may be collected according to conventional techniques and used directly in the method of the invention or stored prior to its use. The sample may be treated prior to its use.

The allele of SNPs may be determined at the level of the DNA, or RNA by any method known by the skilled person.

These methods include, but are not limited to, direct sequencing-based methods, hybridization-based methods, primer extension-based methods, ligation-based methods, methods based on the conformation of a molecule containing the polymorphism, or invasive cleavage-based methods.

In a particular embodiment, the allele of SNPs is detected by direct sequencing using well-known techniques, preferably using next-generation sequencing technologies. The sequencing may be performed on the complete sequence of the TRAIL/TNFS10 gene, or more preferably on the genomic region containing rs3815496 and/or rs1131532.

In another embodiment, the allele of SNPs is detected by selective hybridization. Hybridization based genotyping methods include, but are not limited to, southern hybridization, genotyping on a microarray, in particular on a SNP microarray (amplification products are analysed by hybridization to target sequences immobilized on a solid support), methods using molecular beacons designed to only hybridize to a specific allele, and dynamic allele-specific hybridization (DASH) assay.

In another embodiment, the allele of SNPs is detected by selective amplification. Amplification may be performed according to various techniques known in the art, such as by polymerase chain reaction (PCR), Touch-down PCR (TD-PCR), droplet-digital PCR (ddPCR), quantitative PCR (q-PCR), ligase chain reaction (LCR), strand displacement amplification (SDA) and nucleic acid sequence based amplification (NASBA). These techniques can be performed using commercially available reagents and protocols. Preferred techniques use allele-specific PCR, tetra-primer ARMS-PCR (Shu et al., 2001, Nucleic Acids Res. 29(17): e88).

The allele of SNPs may also be determined by enzyme based methods including, but not limited to, restriction fragment length polymorphism (RFLP) analysis, invader assay relaying on the activity of flap endonuclease that cleaves at specific nucleic acid structures, enzymatic digestion, TaqMan assay or 5'-nuclease probe assay relying on the 5'-nuclease activity of Taq DNA polymerase (e.g. implemented using droplet-digital PCR (ddPCR) or quantitative PCR (q-PCR)); oligonucleotide ligation assay, ligation rolling circle amplification or L-RCA (Xiaoquan et al., 2001, Nucleic Acids Res. 29(22): e116), or primer extension based methods relying on the hybridization of a probe to the bases immediately adjacent to the specific polymorphic allele.

The allele of SNPs may also be determined using methods relying on the differences in conformation, weight, or size of molecules such as PCR-SSCP, MALDI-TOF mass spectrometry (Griffin and Smith, 2000, Trends in Biotechnology, 18: 77-84), denaturing-HPLC and temperature gradient gel electrophoresis (TGCE).

The various methods may be carried out in various reaction formats including homogeneous reactions and reactions on solid supports. Various detection methodologies may be employed in detecting SNPs including, but not limited to, radioactive detection, luminescence detection, fluorescence detection, time-resolved fluorescence detection, fluorescence resonance energy transfer, fluorescence polarization, gel electrophoresis, and mass spectrometry.

Preferably, the allele of SNPs is determined using at least one nucleic acid probe hybridizing to a specific allele.

In a particular embodiment, the allele of the SNP rs1131532 is detected using a nucleic acid probe specifically hybridizing to the C allele and/or a nucleic acid probe specifically hybridizing to the T or G allele, at the polymorphic site rs1131532. Preferably, the allele at the polymorphic site rs1131532 is determined with a 5'-nuclease probe assay, using a nucleic acid probe specifically hybridizing to the C allele and/or a nucleic acid probe specifically hybridizing to the T or G allele, in particular using any probe described below as part of the kit of the invention.

In another particular embodiment, the allele of the SNP rs3815496 is detected using a nucleic acid probe specifically hybridizing to the A allele and/or a nucleic acid probe specifically hybridizing to the G allele, at the polymorphic site rs3815496. Preferably, the allele at the polymorphic site rs3815496 is determined with a 5'-nuclease probe assay, using a nucleic acid probe specifically hybridizing to the A allele and/or a nucleic acid probe specifically hybridizing to the G allele, in particular using any probe described below as part of the kit of the invention.

In another aspect, the present invention also relates to a kit comprising
(i) at least one nucleic acid probe capable of specifically binding or hydridizing to a SNP as disclosed herein, and/or
(ii) at least one set of primers suitable for use in amplification reaction, wherein the set of primers amplifies a SNP as disclosed herein,
and optionally, a leaflet providing guidelines to use such a kit.

The set of primers may allow amplification of the TRAIL/TNFSF10 gene in its entirety, or only a fragment of a SNP as disclosed herein.

In some embodiments, the nucleic acid probes may be attached to at least one reporter molecule generating a detectable signal. The detectable signal may be for example a fluorescent, luminescent or colored signal.

In preferred embodiments, the nucleic acid probes are attached to a fluorophore-quencher pair. These probes are particularly useful in 5'-nuclease probe assays. 5'-nuclease probes are single-stranded hybridization probes labeled with a donor-acceptor fluorophore pair that interact via FRET. The probe is designed to hybridize to its target DNA strand at the same time as the PCR primer. When Taq DNA polymerase extends the primer, it encounters the probe, and as a result of its 5'-nuclease activity, it cleaves the probe. Cleavage of the probe results in the separation of the donor fluorophore and quencher molecule, and leads to an increase in the intensity of the fluorescence signal.

A wide variety of fluorophore-quencher pairs are detailed in the literature (see .e.g. Marras et al. Methods Mol Biol. 2006;335:3-16) and in supplier's catalogues.

Examples of fluorophores include, but are not limited to, fluorescein derivatives such as 5-carboxyfluorescein (5-FAM), 6-carboxyfluorescein (6-FAM), tetrachlorofluorescein (TET) and hexachlorofluorescein (HEX), cyanine dyes such as Cy2, Cy3 and Cy5, Tetramethylrhodamine (TMR), Oregon Green dyes and Texas Red.

Examples of quenchers include, but are not limited to, DABCYL (4-(4-(dimethylamino) phenylazo) benzoic acid), QSY™ quenchers (Invitrogen, Carlsbad, CA), ECLIPSE™ quenchers (Epoch Biosciences, Bothell, WA) and BHQ™ (BHQ-I, 2, and 3 (Biosearch Technologies, Inc. Novato CA)) and DDQ-1 or -2.

In particular embodiments, the fluorophore-quencher pair is selected from HEX-BHQ1 and 6-FAM-BHQ1. Preferably, distinct fluorophore-quencher pairs are attached to each nucleic acid probe type, i.e. probes specific to a first allele and probes specific to the other allele for a specific SNP as disclosed herein, in order to distinguish their signals.

In particular, the nucleic acid probe specific to a first allele of a SNP as disclosed herein may comprise hexachlorofluorescein attached to its 5' end and BHQ1 quencher attached to its 3' end. The nucleic acid probe specific to the other allele of the SNP may comprise 6-carboxyfluorescein attached to its 5' end and BHQ1 quencher attached to its 3' end.

The kit may also comprise at least one molecular beacon or a microarray that can be used to detect a SNP as disclosed herein.

The kit may further comprise one or several reagents for detecting the hybridization of said at least one nucleic acid probe and/or for amplifying and/or detecting said genetic alteration or polymorphism site. In a particular embodiment, the kit further comprise reagents needed to perform droplet digital PCR.

The present invention also relates to the use of this kit for assessing the risk of developing adverse effects after radiotherapy in a subject, said adverse effects being acute and/or subacute dermatitis, and/or lymphocyte apoptosis, or for determining the radiation dose suitable for a subject, according to the method of the invention.

All embodiments disclosed above for the method of the invention are also contemplated in this aspect.

The present invention also takes advantages of the TRAIL modulation.

Further aspects and advantages of the present invention will be described in the following examples, which should be regarded as illustrative and not limiting.

### EXAMPLES

### RESULTS

### TRAIL/TNFSF10 expression correlates with radiosensitivity in T4EM

The sensitivity of subpopulations of human T-lymphocytes to ionizing radiation-induced apoptosis was quantified eighteen hours after irradiation (0-2 Gy) of PBMC samples of healthy blood donors using the previously defined radiosensitivity assay based on immunophenotyping and AnnexinV-labeling. Whereas the CD62L-positive T lymphocyte subpopulations did not undergo apoptosis (Fig. 1), a dose-dependent increase of apoptosis was evidenced in the CD62L-negative T4EM-lymphocytes (Fig. 2 A). Exponential regression coefficients of dose-survival curves were used to classify human PBMC samples according to the radiosensitivity of their T4EM lymphocyte subpopulation, and "sensitive" and "resistant" samples were defined at the two ends of the T4EM radiosensitivity phenotype distribution. To identify genes differentially expressed, array-based expression profiling of flow sorted T4EM-lymphocytes of four "sensitive" and four "resistant" day-fresh samples was performed (Fig. 2 B; black circles). Using a "sensitive" to "resistant" ratio of more than 2 (or less than 0.5), an expression level at least 3 times over background and p<0.01, the inventors identified 31 genes expressed at a higher level (mean DF 2.40; 2.01 to 4.42), and 33 genes expressed at a lower level (mean DF -2.67; -2.01 to -7.48) in "sensitive" samples. Ontology analysis identified three significant pathways, cytokine-cytokine receptor interaction (hsa04060), immune response (GO:0006955), and death (GO:0016265). Among the genes up-regulated in "sensitive" samples, *TRAIL*/*TNFSF10,* displayed a 2.34-fold higher expression level, and was further studied. *TRAIL*/*TNFSF10* mRNA expression levels in flow sorted T4EM lymphocytes of "sensitive", "median" and "resistant" samples (respectively illustrated by "red", "green" and "blue" dots in the distribution of radiosensitivity phenotypes in Fig.2 B) showed that *TRAIL*/*TNFSF10* mRNA level was 5.7-fold higher in "sensitive" compared to "resistant", whereas *TRAIL*/*TNFSF10* mRNA levels of samples with "median" radiosensitivity were intermediate (Fig. 2 C). Only 1 out of 45 studied samples showed discordant T4EM lymphocyte radiosensitivity and *TRAIL*/*TNFSF10* expression level.

To study the effects of irradiation on TRAIL- and TRAIL-receptors mRNA levels in T4EM lymphocytes, the inventors performed multiplexed determination of their mRNA levels on independent triplicate sorts of T4EM lymphocytes from "sensitive" and "resistant" samples at 2, 4, 6 and 24 hours after 2 Gy irradiation. Expression of the decoy receptor *TNFRSF10c*/*DcR1* was below detection limit in T4EM lymphocytes (data not shown). No significant effect of irradiation on *TRAIL*/*TNFSF10,* on decoy receptor *TNFRSF10d*/*DcR2* (not shown), and on pro-apoptotic receptor *TNFRSF10a*/*DR4* mRNA levels was found in "sensitive" or "resistant" samples (Fig. 2 D, left and middle panels). In contrast, in both "resistant" as well as in "sensitive" samples, mRNA levels of the pro-apoptotic receptor *TNFRSF10b*/*DR5* started to increase 4 hours after irradiation and remained high at 4, 6 and 24 hours after irradiation (Fig. 2 D, right panels). This increase in *TNFRSF10b*/*DR5* expression level is in agreement with previous studies that have shown the importance of this pro-apoptotic receptor in radiation-induced apoptosis in mice and in other cell types (Coureuil et al., 2010, PLoS ONE. 5:e12134; Luce et al., 2009, Carcinogenesis. 30:432-439; Finnberg et al., 2005, Mol. Cell. Biol. 25:2000-2013) and the present results suggest that TRAIL in resting T4EM lymphocytes mediates apoptosis by binding to the radiation-induced pro-apoptotic DR5-receptor.

### Radiosensitivity of T4EM depends on mTRAIL expression

TRAIL can be soluble (sTRAIL) or membrane-bound (mTRAIL)(Schneider et al., 1998, J. Exp. Med. 187:1205-1213; Wajant et al., 2001, Oncogene. 20:4101-4106). Under the experimental conditions used for radiation-induced apoptosis of T4EM lymphocytes, the levels of sTRAIL in media were below sandwich ELISA detection level (<20pg/ml; data not shown). The inventors thus focused their study on mTRAIL. Using a monoclonal antibody panel comprising CD253, they measured the level of cell-surface mTRAIL expression by flow cytometry. A four-fold higher expression of mTRAIL was found on T4EM lymphocytes of "sensitive" compared to "resistant" samples (Fig. 3 A). To characterize the role of mTRAIL in radiation-induced apoptosis in T4EM lymphocytes, "sensitive" and "resistant" samples were irradiated in the presence of a soluble blocking antibody to human TRAIL (CD253; clone RIK-2). Apoptosis of T4EM lymphocytes in "resistant" samples was unaffected by the presence of the blocking antibody, whereas blocking of TRAIL resulted in strong inhibition of radiation-induced apoptosis in T4EM lymphocytes in "sensitive" samples, with levels of apoptosis becoming similar to those observed in "resistant" samples, even after a 2.0 Gy irradiation (Fig. 3 B and 3 C).

A ten-fold increase in cell concentration did not increase apoptosis-induction, irrespective of sample sensitivity-classification. On a set of 9 "sensitive" samples, a ten-fold higher cell concentration even resulted in 1.5-fold lower apoptosis-induction in T4EM lymphocytes (Fig. 3 D). These results indicated an autocrine mode of action of mTRAIL, a mode of action that could be abolished by the addition of recombinant human soluble TRAIL (rh-sTRAIL) (Fig. 3 E and 3 F).

### Metalloproteases regulate mTRAIL level in T4EM lymphocytes

Shedding of mTRAIL depends on metalloprotease activity. The inventors thus studied the effect of 1,10-phenanthroline, a broad-range metalloprotease-inhibitor (Yan et al., 2011, CORD Conference Proceedings. 108-111). Two hours after addition of 1,10-phenanthroline, an increase of mTRAIL expression was observed in T4EM lymphocytes in "resistant" and "sensitive" samples (Fig. 4 A and 4 B). However, the level of mTRAIL in T4EM lymphocytes in 1,10-phenanthroline treated "resistant" samples always remained lower than that in untreated "sensitive" samples (Fig. 4 B), suggesting that the limiting factor in mTRAIL presentation is *TRAIL*/*TNFSF10* gene expression.

The increased mTRAIL expression, that was associated with an increased expression of proapototic TRAIL-receptor DR5 (data not shown), was associated with a rapid induction of apoptosis (Fig. 4 C), which could be inhibited by rh-sTRAIL or recombinant human soluble DR5 receptor (rh-sDR5) (Fig. 4D). Interestingly, a significant correlation between the sensitivity of T4EM lymphocytes to 1,10-phenanthroline induced apoptosis and their radiosensitivity was observed (Fig. 4 E). Thus, radio-induced apoptosis in T4EM lymphocytes depends on mTRAIL cell surface expression, which is regulated by metalloprotease activity.

### Genetic association between the TRAIL/TNFSF10 locus and radiosensitivity of T4EM-lymphocytes

To investigate any genetic association between the *TRAIL*/*TNFSF10* locus and radiosensitivity of T4EM lymphocytes, selected regions in the coding- and flanking regions of the *TRAIL*/*TNFSF10* gene that contain SNPs that might be associated to- or act as eQTLs, were genotyped. A 4.2 kb region encompassing the *TRAIL*/*TNFSF10* gene from 373 individuals previously characterized for the T4EM lymphocytes radiosensitivity and subjected to heritability and segregation analysis (Schmitz et al., 2007, Int J Radiat Oncol Biol Phys. 68:1169-1177) was sequenced. Table 1 summarizes results for the 36 polymorphic markers identified, with their frequencies and positions in the studied population. Single marker association analysis between the 15 identified frequent variants (MAF>5%) and T4EM lymphocytes radiosensitivity by Family-Based Association Testing (FBAT) showed a significant association for 3 SNPs; rs3815496 (p=0.03; intron 3, MAF=0.28), rs1131532 (p=0.04; exon 5, MAF=0.28), and rs1131535 (p=0.05; 3' UTR exon 5, MAF=0.38). SNPs rs1131532 and rs3815496 were in strong linkage disequilibrium, and in partial linkage with rs1131535. Several other SNPs in the sequenced region were found to be marginally but significantly associated (Table 2). Multi-Marker association testing between the 15 most frequent SNPs of the *TNFSF10*/*TRAIL* gene and T4EM lymphocytes radiosensitivity was shown significant by the Family based MM association test (FBAT-MM) (p=0.02) and by Fisher product (p=0.04), demonstrating a genetic contribution to the T4EM lymphocytes radiosensitivity in or close to the *TRAIL*/*TNFSF10* gene. The analysis of a selection of 126 unrelated individuals, using T4EM lymphocytes radiosensitivity median values by genotype at rs3815496 identified A as the risk allele for high levels of radiation-induced apoptosis in T4EM lymphocytes (Fig. 5).

**Table 1 : SNP positions and frequencies identified by resequencing in exons and flanking regions of TNFSF10 gene (NM_003810) in the studied population**

| **Position** | **A1** | **A2** | **A2 Frequency (%)** | **dbSNP number** | **Location** | **Frequency class** |
|---|---|---|---|---|---|---|
| 336 | T | C | 4,6 | rs75278014 | Promoter | Rare |
| 380 | C | T | 15,1 | rs12488654 | Promoter | Frequent |
| 404 | A | G | 13,9 | rs365238 | Promoter | Frequent |
| 465 | A | G | 0,2 | rs149647745 | Promoter | Rare |
| 511 | A | G | 15,1 | rs3136586 | Promoter | Frequent |
| 536 | A | C | 1,6 | rs146334252 | Promoter | Rare |
| 1027 | A | G | 0,44 | rs80208847 | Exon (5'UTR) | Rare |
| 1200 | C | T | 0,44 | rs41308132 | Exon 1 (non-synonymous) | Rare |
| 1271 | A | C | 14,6 | rs2270418 | Intron 1 | Frequent |
| 9365 | C | T | 0,35 | rs16845759 | Intron 1 | Rare |
| 9490 | C | A | 0,17 | rs16845759 | Exon 2 | Rare |
| 9871 | A | T | 1,3 | rs142625844 | Intron 2 | Rare |
| 12399 | C | A | 18,9 | rs2241063 | Intron 2 | Frequent |
| 12609 | C | T | 0,17 | rs146586741 | Intron 2 | Rare |
| 12656 | C | T | 0,26 | rs55762319 | Intron 2 | Rare |
| 12969 | C | T | 2,77 | rs3136595 | Intron 3 | Rare |
| 13726 | C | A | 18,9 | rs3136597 | Intron 3 | Frequent |
| 15071 | A | G | 28,13 | rs3815496 | Intron 3 | Frequent |
| 17499 | T | A | 28,25 | rs17848019 | Intron 4 | Frequent |
| 17534 | C | T | 0,61 | rs114244201 | Intron 4 | Rare |
| 17655 | T | C | 0,87 | rs56119116 | Exon 5 (non-synonymous) | Rare |
| 17868 | C | T | 0,26 | rs145496528 | Exon 5 (non-synonymous) | Rare |
| 17967 | C | T | 28,21 | rs1131532 | Exon 5 | Frequent |
| 18089 | C | T | 0,17 | rs138020036 | Exon 5 (3'UTR) | Rare |
| 18192 | A | G | 1,78 | rs41309772 | Exon 5 (3'UTR) | Rare |
| 18195 | G | A | 37,63 | rs1131535 | Exon 5 (3'UTR) | Frequent |
| 18288 | A | G | 4,66 | rs17600346 | Exon 5 (3'UTR) | Rare |
| 18344 | C | A | 28,22 | rs1131542 | Exon 5 (3'UTR) | Frequent |
| 18580 | G | A | 28,09 | rs1131568 | Exon 5 (3'UTR) | Frequent |
| 18643 | G | A | 28,22 | rs1131579 | Exon 5 (3'UTR) | Frequent |
| 18650 | C | T | 28,13 | rs1131580 | Exon 5 (3'UTR) | Frequent |
| 18767 | C | T | 0,51 | rs115111869 | Exon 5 (3'UTR) | Rare |
| 18783 | A | G | 0,43 | rs150169078 | Exon 5 (3'UTR) | Rare |
| 18894 | G | A | 28,51 | rs11720451 | 3' flanking | Frequent |
| 18900 | C | T | 0,09 | rs138626414 | 3' flanking | Rare |
| 18921 | C | T | 0,36 | rs149250622 | 3' flanking | Rare |

**Table 2: Single point association analyses (FBAT) between 15 frequent SNPs of TRAIL/TNFSF10 gene and radiosensitivity of human T4EM lymphocytes.**

| Location | SNP | *p* |
|---|---|---|
| Chr3:172,241,890 | rs12488654 | 0.08 |
| Chr3:172,241,866 | rs365238 | 0.35 |
| Chr3:172,241,759 | rs3136586 | 0.08 |
| Chr3:172,240,999 | rs2270418 | 0.08 |
| Chr3:172,229,871 | rs2241063 | 0.11 |
| Chr3:172,228,544 | rs3136597 | 0.24 |
| Chr3:172,227,199 | rs3815496 | 0.03 |
| Chr3:172,224,771 | rs17848019 | 0.06 |
| Chr3:172,224,303 | rs1131532 | 0.04 |
| Ghr3:172,224,075 | rs1131535 | 0.05 |
| Chr3:172,223,926 | rs1131542 | 0.06 |
| Chr3:172,223,690 | rs1131568 | 0.09 |
| Chr3:172,223,627 | rs1131579 | 0.11 |
| Chr3:172,223,620 | rs1131580 | 0.09 |
| Chr3:172,223,376 | rs11720451 | 0.09 |

### Genetic association of TRAIL/TNFSF10 SNPs rs1131532, rs3815496, and rs1131535 with CD8⁺ lymphocyte apoptosis and with breast cancer radiotherapy induced skin toxicity

Association between the identified *TRAIL*/*TNFSF10* SNPs and CD8⁺ lymphocyte apoptosis as well as with clinical radiosensitivity endpoints was studied in a set of 113 genotyped breast cancer patients, included in the Co-Ho-RT study (Azria et al. 2010, The Lancet Oncology, 11(3), 258-265). Blood samples from these patients are characterized for radiation-induced CD8⁺ T-lymphocyte apoptosis, according to Ozsahin et al., 2005 (Clin Cancer Res. 11:7426-7433). Genetic association study indicated a trend towards association of the three SNPs, rs3815496 (MAF=0.28, p=0.06), rs1131532 (MAF=0.28, p=0.06), and rs1131535 (MAF=0.44, p=0.09), with radiation-induced CD8⁺ T-lymphocyte apoptosis. Finally, acute and subacute dermatitis (10 cases), acute erythema (44 cases), acute hyperpigmentation (7 cases), late fibrosis (28 cases), and late telangiectasia (3 cases) were evaluated for association with these three *TRAIL*/*TNFSF10* SNPs. None of these three SNPs was associated with late fibrosis, acute erythema, or acute hyperpigmentation (Table 3). An association was shown with late telangiectasia (Table 3) but, due to insufficient number of cases this result will not be considered. Interestingly, for acute and subacute dermatitis, a strong association was identified with rs3815496 (p=0.047, effect allele=A, OR: 3.88, 95% c.i: 0.87-17.22), and with rs1131532 (p=0.045, effect allele=C, OR 3.90, 95% c.i: 0.88-17.30), but not with rs1131535.

**Table 3: Association testing (SNPTEST) between identified TRAIL/TNFSF10 SNPs, CD8⁺ lymphocyte apoptosis and radiotherapy-induced skin reaction in a set of 113 genotyped breast cancer patients, included in the Co-Ho-RT study.**

| **Endpoint** | **rs1131535 (effect allele=A, MAF=0.44)** | **rs1131532 (effect allele=C, MAF=0.28)** | **rs3815496 (effect allele=A, MAF=0.28)** |
|---|---|---|---|
| **CD8+ lymphocyte apoptosis** | 1.84 (-0.24,3.93) | 2.09 (-0.09, 4.27) | 2.08 (-0.10, 4.26) |
| | p=0.086 | p= 0.063 | p=0.064 |
| | | | |
| **Acute & subacute dermatitis** | 1.5 (0.63,3.96) | 3.90 (0.88,17.30) | 3.88 (0.87,17.22) |
| (10 cases, 103 controls) | p=0.336 | p= 0.045 | p= 0.047 |
| | | | |
| **Acute Erythema** | 0.78 (0.45,1.34) | 0.69 (0.39,1.25) | 0.69 (0.38,1.24) |
| (44 cases, 69 controls) | p=0.364 | p=0.252 | p=0.244 |
| | | | |
| **Acute Hyperpigmentation** | 1.71 (0.57,5.09) | 1.49 (0.40,5.52) | 1.48 (0.40,5.50) |
| (7 cases, 106 controls) | p=0.338 | p=0.560 | p=0.566 |
| | | | |
| **Late Fibrosis** | 1.10 (0.84, 1.43) | 1.00 (0.76, 1.31) | 1.00 (0.76, 1.31) |
| (28 cases, 83 controls) | p=0.486 | p=0.987 | p=0.975 |
| | | | |
| **Late Telangiectasia** | 6.61(0.76,57.56) | 2.02 (0.23,17.67) | 2.01 (0.23,17.59) |
| (3 cases, 108 controls) | p=0.045 | p=0.517 | p=0.521 |

Advances in radiotherapy treatment suffer from the lack of validated tests and genetic markers that can be used to predict individual radiosensitivity. Human T4EM lymphocyte radiation induced apoptosis indicates that expression of *TRAIL*/*TNFSF10* in resting T4EM lymphocytes identifies individuals with a high apoptotic response to ionizing radiation of these lymphocytes *in vitro.* This T4EM lymphocytes sensitivity can be modulated by anti-TRAIL/CD253 as well as by exogenous sTRAIL, and T4EM lymphocytes apoptosis involving TRAIL signaling could be induced *in vitro* by inhibition of matrix-metalloproteases. Finally, the inventors identified the association between three SNPs within the *TRAIL*/*TNFSF10*-gene and T4EM lymphocytes radiosensitivity, a quantitative trait that had been previously shown to display significant heritability and the segregation of which showed compatible with a Mendelian mode of transmission. A role of TRAIL in the clinical response to radiation and in its physiopathology is suggested by the association between the alleles related to higher apoptosis for two of these three *TRAIL*/*TNFSF10* SNPs and acute and subacute dermatitis, a subset of radiotherapy induced skin reactions (RISR) in a cohort of breast cancer patients. Amongst the identified SNPs, rs1131532 was previously described to be associated with the concentration of the systemic inflammatory biomarker P-selectin (Schnabel et al. 2009, Circulation: Cardiovascular Genetics, 2(3), 229-237). The inventors did not identify association of *TRAIL*/*TNFSF10* SNPs with acute erythema, or with acute hyper-pigmentation.

The inventors did not detect association between the listed TRAIL/TNFSF10 SNPs and late fibrosis, although a trend exists between the *TRAIL*/*TNFSF10* SNPs and the CD8⁺ T-lymphocyte apoptosis phenotype according to Ozsahin. Late fibrosis is a severe complication of radiotherapy that has been previously related to lymphocyte resistance to apoptosis. Association between low apoptosis of CD8 lymphocytes and late fibrosis was first evidenced by Ozsahin et *al.* (Ozsahin et al., 2005, Clin Cancer Res. 11:7426-7433) and replicated in several independent studies. The radiosensitivity phenotyping strategy employed in the present study is markedly different in that it relies on L-Selectin/CD62L-negative CD4 positive lymphocytes only, specifically excluding apoptosis-resistant L-Selectin-positive lymphocytes. It may be relevant to study inter-individual differences in L-selectin shedding/down regulation following radiation to further investigate the association between lymphocyte apoptosis, radiosensitivity, and late toxicity. Altogether the present study highlights a function of TRAIL in lymphocyte radiation induced apoptosis associated with *TRAIL*/*TNFSF10* SNPs. International consortium research that will provide the power of GWAS will enable to appreciate the relative contribution of the identified *TRAIL*/*TNFSF10* SNPs to radiotherapy toxicity (Kerns et al., 2013, Radiother Oncol. 107:372-376).

### METHODS

### Healthy blood donors

All samples were prepared from venous blood drawn from healthy normal volunteers between 9:00 am and 11:00 am. The inclusion of healthy volunteers was preceded by a medical interview and informed written consent was obtained from all donors, in accordance with local ethics guidelines at Fondation Jean Dausset-CEPH or Etablissement Français du Sang, Hôpital Saint Louis, Paris.

### Samples

Peripheral blood mononuclear cells (PBMC) were prepared from fresh (within 6h, average 180 minutes) anticoagulated blood samples, by standardized procedures. Briefly, 30 ml of two-fold diluted blood were underlayered with 10 ml of Histopaque^{®}-1077 (Sigma-Aldrich), and centrifuged at 2,000g for 20 minutes. The collected interlayer was washed twice with 40 ml of RPMI1640/15% Fetal Calf Serum (FCS), and used as further described or collected in FCS/10% DMSO for cryopreservation. Fractions of 1 ml each, corresponding to 4 ml of blood, were immediately frozen, using controlled temperature decrease, and stored in liquid nitrogen. Thawing of cells was performed by rapid transfer of cryotubes to 56 °C waterbath (45 sec), addition of 1 ml of FCS and transfer of contents to 12 ml of RPMI/15% FCS. Thawed cells were washed twice, to be finally resuspended in the appropriate volume of culture medium (RPMI1640/15% FCS).

DNA extractions were performed from PBMC by classical phenol/chlorophorm procedures. Yield and purity were assessed spectrophotometrically at 260 and 280 nm (Thermo Scientific NanoDrop(TM) 1000). Familial relations were recorded from oral interview and HLA-A sequencing was used to exclude samples presenting incompatibilities with announced familial interrelations from family based association analysis.

### Monoclonal antibodies and proteins

All fluorochrome-coupled monoclonal antibodies were used at saturating conditions, as determined by titrations. Anti-human CD antibodies came from Becton Dickinson ImmunoCytometry Systems (CD62L-PE, CD62L-PECy5.1, CD14-PECy5.1, CD-235a- PECy5.1, CD27-PECy5.1, CD4-PECy7, CD19-PECy7, CD45RA-APC, CD8-APCH7), from Beckman Coulter (CD3-PETxR) from Biolegend (CD253/TRAIL-PE, CD62L- BV421, CD3-BV605), or from R&D Systems (CD262/DR5-APC). Recombinant human soluble TRAIL (rh-sTRAIL) and recombinant human soluble DR5 (rh-s-TRAIL was from R&D Systems, AnnexinV-FITC from Becton Dickinson, and 1,10-phenanthroline from Sigma-Aldrich.

### Apoptosis induction

Cell suspensions at approximately 5x10⁵ cells/ml (or at cell densities specified in the results section) in RPMI1640/15% FCS, were irradiated at a dose rate of approximately 0.5 Gy/min with a ¹³⁷Cs-source (IBL637; Cis-Bio international/Scherring, Saclay, France), where incubated in the presence of indicated concentrations of recombinant human soluble TRAIL (rh-sTRAIL), recombinant human soluble DR5 (rh-sDR5), anti-TRAIL cytotoxicity-blocking antibody (CD253; clone RIK-2), or of 1,10-phenanthroline (stock solution of 200 mM in DMSO). All experiments described were performed with the same batch of heat inactivated FCS, previously tested for compatibility with our experimental procedures. Incubations of 18 hours or less as specified in results section were at 37 °C and 5% CO₂.

### Cell labeling for lymphocyte subset identification and apoptosis quantification

Treated cells were collected and washed with AnnexinV buffer (140mM NaCl, 5mM CaCl₂, 10mM HEPES; ph7.4), and stained with 2.5 µl AnnexinV-FITC in combination with fluorochrome-conjugated CD markers in 100ul for 30 min. For cell sorting for gene expression analysis, Annexin V positive cells were excluded, and CD3 labeling was omitted to avoid TCR-mediated activation signaling. Furthermore, CD14 and CD235a were included to exclude monocytes and reticulocytes susceptible to contaminate the sort gates. CD27 was included for sorts of cryopreserved samples to avoid contamination of sort gates by central memory lymphocytes that transiently lost CD62L through sample manipulation procedures. For CD253 (mTRAIL) quantification, a compact panel designed to limit the need for compensation including AnnexinV, CD253, CD3, CD4, and CD62L was used.

### Flow Cytometry

Cells were washed in AnnexinV-buffer and just prior flow cytometry, 50 µL of AnnexinV-buffer containing 0.2 µg/mL Hoechst 33258 (Molecular Probes, Eugene, OR), was added for dead cell exclusion. Samples were analyzed using a three-laser CyAn LX system, (DakoCytomation, Fort Collins, CO) or a 5-laser SORP LSRII (Becton Dickinson). Cell sorting was performed using a custom-built five-laser InFlux system (Becton Dickinson). All filters were from Chroma Technologies Inc., Brattleboro, Vermont All fluorescence signals were log-amplified, and stored in listmode. CompBeads (Becton Dickinson) were used to assist in determination of spill-over in every individual experiment (CD3-FITC was used instead of AnnexinV-FITC) and Fluorescence Minus One (FMO) controls were used where needed, in particular for mTRAIL quantifications (SortWare; Becton Dickinson or Flowjo version 9.6.4 ; Treestar, Ashland, OR). All sorts were performed in single droplet count mode, with an extended coincidence mask of one-and-a-half droplet. Doublet exclusion was performed by gating on the triggering parameter (FSC) versus time of flight.

### Data analysis

Data was analyzed using FlowJo. Listmode file analysis proceeded through the exclusion of non-lymphocyte events, predominantly monocytes, by using a large scattergate, shaped to include apoptotic *lymphocytes* (lower FSC, higher SSC). Subsequently, dead cells were excluded on the basis of HO33258 fluorescence. T Lymphocytes were identified on the basis of positive PETxR (CD3) fluorescence. A PECy7 (CD4) versus APCH7 (CD8) histogram allowed identification of CD4-positive T *lymphocytes* (T4) and CD8-positive T *lymphocytes* (T8). An APC (CD45RA) versus PE (CD62L) histogram discriminated between naive (CD62L+CD45RA+), central memory (CM; CD62L+CD45RA-), effector memory (EM; CD62L-CD45RA-), and terminal effector (TE; CD62L- CD45RA+) T4 and T8. The proportion of apoptotic cells was determined by application of an identical gate on a bivariate plot of FSC versus FITC fluorescence (AnnexinV) to all of the identified subpopulations. Dose-effect curves were generated using at least four doses, and used for the quantitative evaluation of 1,10-phenanthroline sensitivity and radiation sensitivity, as previously described (Schmitz et al., 2003, Int J Radiat Oncol Biol Phys. 57:769-778).

### Microarray cDNA hybridization analysis

Cells of interest, recovered by centrifugation directly after sort, were lysed by RLT buffer (Qiagen) and cryopreserved until RNA extraction. Total RNA was extracted from a minimum of 300,000 cells using the RNeasy Plus Micro Kit (Qiagen). RNA samples were re-suspended in RNase-free water and stored at -80°C. RNA yield and purity were assessed spectrophotometrically at 260 and 280 nm (Thermo Scientific NanoDrop(TM) 1000). RNA integrity was evaluated using a Bioanalyzer 2100 (Agilent). Microarray experiments and part of data analysis were performed by PartnerChip (S. B., Evry, France) following the Affymetrix-recommended procedures. Target was prepared from 4 sensitive and 4 resistant individuals. Targets were pooled after individual quality control in equal proportions and hybridized on a HU133 2.0 plus array (54645 probe sets) according to the Affymetrix Two-Cycle technical protocol. Fluorescent images were detected in a GeneChip Scanner 3000 (Affymetrix). Expression data and raw expression data (CEL files) were generated using GCOS software (Affymetrix). Quality control was assessed based on 3'/5' ratios of glyceraldehyde 3-phosphate dehydrogenase (GAPDH) and β-actin control probe sets.

### Real-time quantitative PCR gene expression analysis

Triplicate sorts for qPCR gene expression studies were performed by direct deposition of cells in MicrAmp optical tubes (Applied Biosystems), containing 15 µl of one step RT-qPCR mix. RT-qPCR pre-amplification mix contained Platinum Taq polymerase and SuperScript III reverse transcriptase (Invitrogen), a mixture of Taqman primer-probes, specific for the transcripts of interest (Table 4; Applied Biosystems) at 0.2X concentration, CellsDirect One-Shot qRT-PCR buffer (Invitrogen), and Superaseln RNase inhibitor. Immediately following cell sorting, samples were thoroughly mixed, and reverse transcribed (55 °C for 10 min, 50 °C for 50 min), and pre-amplified (14 cycles of PCR at 95 °C for 15 sec and 62 °C for 45 sec). After pre-amplification, samples were diluted ten-fold, for gene-specific quantitative PCR (35 cycles of 95 °C/15 sec, 62 °C/45 sec) on a HT7900 (Applied Biosystems). All primers were standard Taqman assays (Applied Biosystems), and reaction conditions were according to manufacturers indications. Results were expressed as the difference between the Ct of the internally amplified reference gene (VIC-labeled *RPLP0, ACTB,* or *GAPDH),* and the FAM-labeled test gene, averaged over triplicate sorts, and termed dCT. Assay performance control on cryopreserved lymphocytes included mean dCt on different cryopreserved vials from same blood sample, mean dCt from independent blood samples from same individual. Samples from reference individuals were included in each experiment. Radiation induction of expression is expressed as the difference between dCT at dose 2.0 Gy and dCt at dose 0 Gy, for each timepoint after irradiation, and was termed ddCT.

**Table 4: List of qPCR TaqMan assays**

| **Gene** | **Reporter** | **AIF Assay ID** |
|---|---|---|
| *ACTB* | VIC | Hs99999903_m1 |
| *GAPDH* | VIC | Hs99999905_m1 |
| *RPLP0* | VIC | Hs99999902_m1 |
| *TNFRSF10a* | FAM | Hs00269492_m1 |
| *TNFRSF10b* | FAM | Hs00366278_m1 |
| *TNFRSF10c* | FAM | Hs00182570_m1 |
| *TNFRSF10d* | FAM | Hs00388742_m1 |
| *TNFSF10* | FAM | HS00234356_m1 |

### DNA genotyping

Primers were established in order to amplify by PCR the exon-containing DNA fragments and the promoters. The PCRs were performed in a 15 µL reaction mixture containing 25 ng of DNA (lists of primers Tables 5 and 6). Sequencing reactions were performed according to the dye terminator method using an ABI PRISM^{®} 3730xl DNA Analyzer (Applied Biosystems, Foster City, CA, USA). Alignment of experimental results, SNP discovery and genotyping were performed with the software Genalys. The genomic sequence used for the alignment is *TRAIL*/*TNFSF10* (NC_000003.11). The genotypes for rs3136597 and rs2241063 were obtained by Taqman technology, assays C_27464917_20 and C_3260973_20 respectively (Applied Biosystems).

**Table 5: List of primers used for genotyping of TNFSF10**

| **PCR Forward primer** | **Sequence** | **PCR Reverse primer** | **Sequence** |
|---|---|---|---|
| TRAILProPF | ACAACATACAGCTGGGCCAG (SEQ ID No 3) | TRAILProPR | GTAGTCGTTGGAAAGGAGGG (SEQ ID No 4) |
| TRAILE1PF | AAACAGGCCTTGTGCCTATG (SEQ ID No 5) | TRAILE1PR | GCTTTCATGAAGAGTTGCAATG (SEQ ID No 6) |
| TRAILE2PF | TGGCAGAACTGGAAGAGACC (SEQ ID No 7) | TRAILE2PR | CATCAGCAATGTGGGAAGAA (SEQ ID No 8) |
| TRAILE3SF | CCACATTTGGCTGACATCAC (SEQ ID No 9) | TRAILE3PR | ATGATGGAGGAGGAGGCTTT (SEQ ID No 10) |
| TRAILE4PF | GTTTCTCTTTGGACCCTACC (SEQ ID No 11) | TRAILE4PR | TTTCCTGAGGCCAGTTATGTC (SEQ ID No 12) |
| TRAILE5-1PF | CTCCCAACAGTTCCCAATGT (SEQ ID No 13) | TRAILES-1PR | CCTTAAGGAAACCTGGAGGC (SEQ ID No 14) |
| TRAILE5-2PF | GGGGCCTTTTTAGTTGGCTA (SEQ ID No 15) | TRAILE5-2PR | CAGCACAACTCAACCCAGAA (SEQ ID No 16) |

**Table 6: List of primers used for exons sequencing TNFSF10**

| **SEQ Forward primer** | **Sequence** | **SEQ Reverse primer** | **Sequence** |
|---|---|---|---|
| | | TRAILE1SR | CCACAGAGAAAGGAAGCAGG (SEQ ID No 20) |
| | | TRAILE2SR | TTACCCTGTGTGTGCCTCAG (SEQ ID No 21) |
| | | TRAILE3SR | TGAGCTTCCTAGCTGCCAAT (SEQ ID No 22) |
| TRAILE5-1SF | TCCTGGGAATCATCAAGGAG (SEQ ID No 17) | TRAILE5-1SR | CCTCCTGAAATCGAAAGTATG (SEQ ID No 23) |
| TRAILE5-1SF2 | GCAGGAACCTCCCAATTTCT (SEQ ID No 18) | | |
| TRAILE5-2SF | AATCTGAGTAGAGCAGCCAC (SEQ ID No 19) | TRAILE5-2SR2 | TGGCATGATCTCACCACACT (SEQ ID No 24) |

### Family Based Association testing of frequent variants (FBAT)

The FBAT method and software (Laird et al., 2000, Genet Epidemiol. 19 Suppl 1:S36-42; Rabinowitz and Laird, 2000, Hum Hered. 50:211-223) was used to perform a single marker analysis between the frequent SNPs and the quantitative trait while taking into account the family structure (command fbat -o). Two methods have been used to obtain a *p*-value for the global association of *TNFSF10* with the trait : a) The Fisher product (Fisher, 1925, Statistical Methods for Research Workers.) of the *p*-values obtained from the single point association analysis was taken, and its significance was assessed using 10,000 permutations, b) The FBAT Multi-Marker statistic (Rakovski et al., 2007, Genet Epidemiol. 31:9-17; Chen et al., 2013, Genet Epidemiol. 37:196-204) was computed, and its significance was assessed using 10,000 permutations.

### Radiotherapy induced skin reaction association testing with TRAIL/TNFSF10 SNPs (SNPTEST)

Acute and subacute dermatitis, acute erythema, acute hyperpigmentation and late fibrosis were assessed prospectively using the CTCAE v3 grading system in the CO-HO-RT trial (Azria et al. 2010, The Lancet Oncology, 11(3), 258-265). Toxicity was treated as a binary outcome and was determined by taking the highest CTCAE grade occurrence during radiotherapy to 6 weeks after radiotherapy for acute toxicities and from 3 months after radiotherapy to the end of follow-up (minimum 2 years, maximum 8 years) for late toxicities. For each toxicity endpoint, patients were considered cases if they had CTCAE grade ≥ 2 and controls if they had CTCAE grade ≤ 1. The percentage of CD8+ lymphocytes undergoing radiation-induced apoptosis was treated as a continuous outcome.

Genomic DNA was isolated from blood, genotyped using a commercial genome-wide SNP array (Affymetrix SNP6.0, Affymetrix, San Diego, CA)]. Because rs1131532, and rs3815496 were not directly genotyped on the array, the surrounding region of chromosome 3 (position 100,000,001 to 200,000,000, hg build 19) was imputed to the 1000 Genomes reference data using IMPUTE2 software [PMID: 19543373]. Following imputation, the SNPs of interest were confirmed to be in Hardy-Weinberg equilibrium (p-value>10⁻⁶) and showed good imputation quality (info score>0.3). SNPTEST software [PMID: 17572673] was used to analyze SNP-phenotype association with the frequentist test and expected method, which uses expected genotype counts (i.e. genotype dosages) obtained from imputation. An additive genetic inheritance model was assumed in all analyses.

### Statistical analysis

Statistical analyses and data presentation were performed with Stata (Stata Corp.,College Station, TX). In box plot representations of results, boxes extend from 25^{th} to 75^{th} percentiles, with whiskers indicating upper- and lower adjacent values (1.5 times interquartile range). Each box represents at least three independent experiments.

## Claims

1. Use of a single nucleotide polymorphism (SNP) selected from the group consisting of rs1131532, rs3815496 and any SNP having a linkage disequilibrium with an absolute value for r² of 0.85 therewith as a biomarker for assessing the risk of developing adverse effects after radiotherapy in a subject, wherein said adverse effects are selected for the group consisting of acute and/or subacute dermatitis and lymphocyte apoptosis.

2. The use according to claim 1, wherein the SNP having a linkage disequilibrium with rs3815496 is selected in one of the following groups consisting of
a) rs1131532, rs9811673, rs9811639, rs3136600, rs3815496, rs3181143, rs3181142, rs3136606, rs3136607, rs3819773, rs1126161, rs63562861, rs1131542, rs1131568, rs11720451, rs9859809, rs6807069, rs6783667, rs9869255, rs9811650, rs9811646, rs62282578, rs1992868, rs1992869, rs6414542, rs1597086, rs9880799, and rs4894557, more preferably from the group consisting of rs3815496, rs3181143, rs3181142, rs3136606, rs3136607, rs3819773, rs1126161, rs63562861, rs1131542, rs1131568, rs11720451, rs9859809, rs6807069, rs6783667, rs9869255, rs9811650, rs9811646, rs62282578, rs1992868, rs1992869, rs6414542, rs1597086, rs9880799, and rs4894557;
b) rs1131532, rs1131542, rs3815496, rs3181143, rs3136600, rs3136606, rs3136607, rs3819773, rs1126161, rs63562861, rs11720451, rs9859809, rs6807069, rs6783667, rs9811673, rs9869255, rs9811650, rs9811646, rs62282578, rs1992868, rs1597086, rs9811639, rs1992869, rs6414542, rs9880799, rs4894557, rs9859259, and rs9880475, more preferably from the group consisting of rs1131532, rs3815496, rs3181143, rs3136600, rs3136606, rs3136607, rs3819773, rs1126161, rs63562861, rs1131542, rs11720451, rs9859809, rs6807069, rs6783667, rs9811673, rs9869255, rs9811650, rs9811646, rs62282578, rs1992868, and rs1597086;
c) rs3136600;
d) rs1131532, rs9811673, rs9811639, rs3136600, rs3815496, rs3181143, rs3136606, rs3136607, rs3819773, rs1126161, rs63562861, rs1131542, rs11720451, rs9859809, rs6807069, rs6783667, rs9869255, rs9811650, rs9811646, rs62282578, rs1992868, rs1992869, rs6414542, rs1597086, rs9880799 and rs4894557.

3. The use according to claim 1, wherein the SNP having a linkage disequilibrium with rs1131532 is selected in one of the following groups consisting of
a) rs3815496, rs1131542, rs63562861, rs1131568, rs11720451, rs9859809, rs3819773, rs3136607, rs3136606, rs6807069, rs6783667, rs9869255, rs9811650, rs9811646, rs62282578, rs3181143, rs3181142, rs1992868, rs1992869, rs6414542, rs1597086, rs9880799, rs1126161, rs4894557, rs9811673 and rs9811639;
b) rs3815496, rs1131542, rs63562861, rs11720451, rs9859809, rs3819773, rs3136607, rs3136606, rs3136600, rs6807069, rs6783667, rs9811673, rs9869255, rs9811650, rs9811646, rs62282578, rs3181143, rs1992868, rs1597086, rs1126161, rs9811639, rs1992869, rs6414542, rs9880799, rs4894557, rs9859259 and rs9880475, more preferably from the group consisting of rs3815496, rs1131542, rs63562861, rs11720451, rs9859809, rs3819773, rs3136607, rs3136606, rs3136600, rs6807069, rs6783667, rs9811673, rs9869255, rs9811650, rs9811646, rs62282578, rs3181143, rs1992868, rs1597086 and rs1126161
c) rs1131542, rs11720451, rs3819773, rs3136607, rs3136606, rs6807069, rs6783667, rs12696330, rs9811673, rs9869255, rs9811650, rs9811646, rs9811639, rs62282578, rs9849142, rs1992868, rs1992869, rs6414542, rs1597086, rs9880799, rs9859809 and rs63562861;
d) rs3815496, rs1131542, rs63562861, rs11720451, rs9859809, rs3819773, rs3136607, rs3136606, rs6807069, rs6783667, rs9869255, rs9811650, rs9811646, rs62282578, rs3181143, rs1992868, rs1992869, rs6414542, rs1597086, rs9880799, rs1126161, rs4894557, rs9811673, and rs9811639.

4. The use according to claim 1, wherein the SNP having a linkage disequilibrium with rs1131532 and rs3815496 is selected from the group consisting of rs9811673, rs9811639, rs3815496, rs3181143, rs3136606, rs3136607, rs3819773, rs1126161, rs63562861, rs1131542, rs11720451, rs9859809, rs6807069, rs6783667, rs9869255, rs9811650, rs9811646, rs62282578, rs1992868, rs1992869, rs6414542, rs1597086, rs9880799 and rs4894557.

5. The use according to claim 1, wherein the SNP is rs3815496 or rs1131532.

6. A method for assessing the risk of developing adverse effects after radiotherapy in a subject, wherein the method comprises determining the allele of a single nucleotide polymorphism (SNP) selected from the group consisting of rs1131532, rs3815496 and any SNP having a linkage disequilibrium with an absolute value for r² of 0.85 therewith in a subject sample; wherein said adverse effects are selected for the group consisting of acute and/or subacute dermatitis and lymphocyte apoptosis.

7. The method according to claim 6, wherein the SNP having a linkage disequilibrium with rs3815496 is selected in one of the following groups consisting of
a) rs1131532, rs9811673, rs9811639, rs3136600, rs3815496, rs3181143, rs3181142, rs3136606, rs3136607, rs3819773, rs1126161, rs63562861, rs1131542, rs1131568, rs11720451, rs9859809, rs6807069, rs6783667, rs9869255, rs9811650, rs9811646, rs62282578, rs1992868, rs1992869, rs6414542, rs1597086, rs9880799, and rs4894557, more preferably from the group consisting of rs3815496, rs3181143, rs3181142, rs3136606, rs3136607, rs3819773, rs1126161, rs63562861, rs1131542, rs1131568, rs11720451, rs9859809, rs6807069, rs6783667, rs9869255, rs9811650, rs9811646, rs62282578, rs1992868, rs1992869, rs6414542, rs1597086, rs9880799, and rs4894557;
b) rs1131532, rs1131542, rs3815496, rs3181143, rs3136600, rs3136606, rs3136607, rs3819773, rs1126161, rs63562861, rs11720451, rs9859809, rs6807069, rs6783667, rs9811673, rs9869255, rs9811650, rs9811646, rs62282578, rs1992868, rs1597086, rs9811639, rs1992869, rs6414542, rs9880799, rs4894557, rs9859259, and rs9880475, more preferably from the group consisting of rs1131532, rs3815496, rs3181143, rs3136600, rs3136606, rs3136607, rs3819773, rs1126161, rs63562861, rs1131542, rs11720451, rs9859809, rs6807069, rs6783667, rs9811673, rs9869255, rs9811650, rs9811646, rs62282578, rs1992868, and rs1597086;
c) rs3136600;
d) rs1131532, rs9811673, rs9811639, rs3136600, rs3815496, rs3181143, rs3136606, rs3136607, rs3819773, rs1126161, rs63562861, rs1131542, rs11720451, rs9859809, rs6807069, rs6783667, rs9869255, rs9811650, rs9811646, rs62282578, rs1992868, rs1992869, rs6414542, rs1597086, rs9880799 and rs4894557.

8. The method according to claim 6, wherein the SNP having a linkage disequilibrium with rs1131532 is selected in one of the following groups consisting of
a) rs3815496, rs1131542, rs63562861, rs1131568, rs11720451, rs9859809, rs3819773, rs3136607, rs3136606, rs6807069, rs6783667, rs9869255, rs9811650, rs9811646, rs62282578, rs3181143, rs3181142, rs1992868, rs1992869, rs6414542, rs1597086, rs9880799, rs1126161, rs4894557, rs9811673 and rs9811639;
b) rs3815496, rs1131542, rs63562861, rs11720451, rs9859809, rs3819773, rs3136607, rs3136606, rs3136600, rs6807069, rs6783667, rs9811673, rs9869255, rs9811650, rs9811646, rs62282578, rs3181143, rs1992868, rs1597086, rs1126161, rs9811639, rs1992869, rs6414542, rs9880799, rs4894557, rs9859259 and rs9880475, more preferably from the group consisting of rs3815496, rs1131542, rs63562861, rs11720451, rs9859809, rs3819773, rs3136607, rs3136606, rs3136600, rs6807069, rs6783667, rs9811673, rs9869255, rs9811650, rs9811646, rs62282578, rs3181143, rs1992868, rs1597086 and rs1126161
c) rs1131542, rs11720451, rs3819773, rs3136607, rs3136606, rs6807069, rs6783667, rs12696330, rs9811673, rs9869255, rs9811650, rs9811646, rs9811639, rs62282578, rs9849142, rs1992868, rs1992869, rs6414542, rs1597086, rs9880799, rs9859809 and rs63562861;
d) rs3815496, rs1131542, rs63562861, rs11720451, rs9859809, rs3819773, rs3136607, rs3136606, rs6807069, rs6783667, rs9869255, rs9811650, rs9811646, rs62282578, rs3181143, rs1992868, rs1992869, rs6414542, rs1597086, rs9880799, rs1126161, rs4894557, rs9811673, and rs9811639.

9. The method according to claim 6, wherein the SNP having a linkage disequilibrium with rs1131532 and rs3815496 is selected from the group consisting of rs9811673, rs9811639, rs3815496, rs3181143, rs3136606, rs3136607, rs3819773, rs1126161, rs63562861, rs1131542, rs11720451, rs9859809, rs6807069, rs6783667, rs9869255, rs9811650, rs9811646, rs62282578, rs1992868, rs1992869, rs6414542, rs1597086, rs9880799 and rs4894557.

10. The method according to claim 6, wherein the SNP is rs3815496 or rs1131532.

11. The method according to any one of claims 6-10, wherein the step of determining the allele is a step of genotyping by sequencing, selective hybridization and/or selective amplification, enzyme-based methods or methods relying on differences in the conformation, weight or size of the molecules.

12. The method according to any one of claims 6-11, wherein the subject sample is saliva, urine, whole blood, plasma, or serum sample.

13. A method for determining the radiation dose suitable for a subject, comprising performing the method according to any one of claims 6-12, and selecting the suitable radiation dose for the subject so as a reduced radiation dose is selected if the subject has at least one allele of the SNP associated with an increased risk of developing adverse effects after radiotherapy and a maximal radiation dose is selected if the subject does not present any allele of the SNP associated with an increased risk of developing adverse effects after radiotherapy.

14. Use of a kit for assessing the risk of developing adverse effects after radiotherapy in a subject, said adverse effects being acute and/or subacute dermatitis and/or lymphocyte apoptosis, or for determining the radiation dose and/or selecting the radiotherapy delivery suitable for a subject, wherein the kit comprises at least one nucleic acid probe capable of specifically binding or hybridizing a SNP as defined in claims 1-5, and/or at least one set of primers suitable for use in amplification reaction suitable for amplifying a SNP as defined in claims 1-5.
